# EUROPEAN PATENT APPLICATION

(11) **EP 4 628 585 A1**
(43) Date of publication of application: **08.10.2025**
(21) Application number: 24168345.7
(22) Date of filing: 03.04.2024
(51) Int. Cl.: C12N 15/113, A61K 31/7088

(54) **ANTISENSE OLIGOMERS AND METHODS FOR TREATING ATOPIC DERMATITIS, PSORIASIS AND OTHER INFLAMMATORY CONDITIONS**

(71) Applicant: Prion OÜ, 50410 Tartu (EE)
(72) Inventor: KÕKS, Sulev, Perth, 6008 (AU); KÕKS, Gea, Perth, 6008 (AU); DUBRAC, Sandrine, 6020 Innsbruck (AT)
(74) Representative: Sarap, Margus

(57) **Abstract**

The present invention describes multiple isolated antisense oligomers with the ability to alter the translation of the genes in the inflammatory network that are activated during the pathogenesis of atopic dermatitis, psoriasis, and other chronic inflammatory skin disorders to support the tissue repair and regeneration by reducing the tissue damage caused by the oxidative stress and the inflammation.

## Description

### TECHNICAL FIELD

The present invention described to structure and use of the antisense oligomers to treat, prevent, or ameliorate the atopic dermatitis, psoriasis or other skin and systemic inflammatory conditions. This invention also supports the tissue regeneration by reducing the tissue damage caused by the oxidative stress and the inflammation.

### BACKGROUND ART

Atopic dermatitis (AD) is the most common inflammatory skin disease, predominantly affecting young children and characterized by relapsing pruritic eczematous lesions over dry skin. AD affects 1-37% of children and 1-20% of adults worldwide and became a major health problem among children born after 1980. Importantly, AD is considered the initial step of the so-called 'atopic march'. Indeed, whereas 70% of children afflicted with AD experience a full remission at 10-12 years of age, as many as 20-70% of them go on to develop asthma, allergic rhinitis and/or food allergies. Moreover, the chronic and relapsing nature of AD significantly impairs the quality of life of patients and their relatives. Affected individuals often have to cope with mental health issues such as depression, attention deficit/hyperactivity disorder or social isolation. AD is a complex disease with multiple factors playing a role in its pathophysiology whose combination leads to a broad spectrum of disease manifestation. AD patients display a rough, dry skin even before initial inflammatory symptoms occur. Once skin lesions develop, they can present as acute, subacute or chronic. The first manifestations of acute AD appear as pruritic, eczematous skin lesions that show an age-related distribution. In infancy, common sites of involvement are the face (cheeks) and the extensor surfaces of the limbs, whereas in older children and adults, lesions are often chronic and mainly localized to the hands, neck and the flexural folds of extremities. AD is a fluctuating disease with episodes of relapse and remission during which symptoms can range from dry scaly skin to severe erythroderma.

Psoriasis is the life-long disease that starts between 15 and 30 years of age. It is one of the most common diseases affecting skin and joints in 2.5% of the world population. It is characterised by red, scaly skin patches with well-defined edges that are usually found on the scalp, elbows, and knees, but can be found in any place of the body. The psoriatic scale is typically with silvery white, but in the skin, folds can appear shiny with the moist peeling surface. Psoriasis can also affect very large regions of the body like chest, abdomen, or entire back of the patient. The typical clinical form of the psoriasis is the generalised chronic plaque psoriasis that affects multiple body regions. Without treatment the plaques are very persistent, do not disappear and long-term skin deformities can develop.

Psoriasis is commonly associated with a painful and deforming arthritis that can cause severe disability of the patient. Psoriatic arthritis can be in the patients with skin plaques, but also in patients without the skin involvement. Psoriatic arthritis usually causes swollen fingers and toes but can also cause pain in the points of tendon attachments to the bones, especially at the back of the heels. Both psoriasis and psoriatic arthritis can cause lower back pain that is a symptom of spondylitis or the inflammation of the joints between the vertebrae of the spine or the joints between the spine and pelvis (sacroiliitis). Psoriasis is also characterised by the nail involvement, psoriatic nails. Typical psoriatic nail damages include tiny dents, crumbling of the nails or even separation of the nails from the nail beds.

Depending on the affected body site, psoriasis can have quite different clinical features and subtypes of the disease. When psoriasis is localised to the soles and palms, the disease is a palmoplantar psoriasis, and this can be isolated as a part of the generalised chronic plaque psoriasis. Palmoplantar psoriasis is characterised by patchy or generalised thickening and scaling of the entire surface of palms or soles without redness. Palms and soles affected by psoriasis can be red, but they always have dry and thickened, deep painful cracks or fissures, that can even bleed. The skin changes are symmetrical, on both palms or soles, and have sharp borders. Palmoplantar psoriasis is chronic and is very persistent to any existing therapies. Palmoplantar psoriasis is more commonly associated with the psoriatic nail dystrophy and psoriatic arthritis.

Another disease with similar features to psoriasis is a palmoplantar pustulosis. This is an uncommon skin condition characterised by the pustular rash that presents as crops of sterile pustules in hands and feet. This rash is associated with thickened, scaly, and red skin that easily develops deep painful fissures and cracks. Palmoplantar pustulosis is a localised form of the generalised pustular psoriasis, that is a life-threatening disease characterised by sudden, repeated episodes of high-grade fever, generalised rash, disseminated pustules, and elevated systemic inflammation markers in the blood.

Generalised pustular psoriasis is commonly together with psoriasis but can be independently.

In AD, the impaired epidermal barrier is, per se, sufficient to enhance keratinocyte hyperproliferation and synthesis of lipid, DNA and protein in an effort to restore the barrier. This response is part of a hierarchical imperative of sustaining a fully competent barrier in a desiccating terrestrial environment and leads to mild acanthosis in non-lesional AD skin. When the skin becomes itchy, scratching potentially enhances the penetration of antigens or bacteria into the skin and also the release of proinflammatory lipids, i.e. eicosanoids. This likely induces a response in keratinocytes leading to the production of inflammatory mediators and the recruitment of immune cells to the skin, thereby advancing the transition from non-lesional to lesional AD.

Psoriatic skin lesions are typical red, scaly patches that are called plaques. These lesions are caused by the excessive proliferation of the keratinocytes and the infiltration by the inflammatory cells. The inflammatory activating signal is excessive and causes uncontrollable proliferation of keratinocytes that are not able to mature. The skin surface is roughened because of excessive and abnormal scale formation. Therefore, the skin's barrier function is severely damaged. Despite the high incidence, treatment options are limited, and psoriasis poses a major challenge to the health care system where most patients do not receive effective and sustainable treatments. The disorder is initiated and maintained by chronic inflammation that has intermittent course with periods of remissions and relapses (flares). Taken together, psoriasis is a chronic inflammatory disorder that primarily affects skin and joints. Disease progression often leads to psoriatic arthritis that are not always accompanied by the structural changes in the skin. However, given the chronic nature of the disease, long lasting inflammation leads to the structural rearrangements of the affected tissues.

Long lasting effects of the atopic dermatitis and psoriasis can lead to the lichenification of the skin that is characterised by thickened leathery skin and increased skin markings. This can lead to the development of skin cracks and deep, painful, even bleeding fissures. When psoriatic plaques clear up, they often leave discoloration (brown or pale) marks that will fade over several months.

AD is associated with cutaneous bacterial, viral and fungal infections, including extracutaneous infections in severe forms. AD is also associated with several mental health comorbidities particularly attention-deficit hyperactivity disorder, anxiety, and depression. Moreover, AD patients are more prone to develop specific cancers, such as adult lymphomas. Furthermore, AD has been shown to be associated with obesity, cardiovascular disease, and autoimmune disease, particularly alopecia areata and gastrointestinal immune-mediated disorders.

The chronic inflammatory nature of the psoriasis generates several comorbidities and health risks for the patients if disease is left unattended or is poorly managed. Psoriasis is a systemic inflammatory disease that is associated with an increased risk for many systemic comorbidities. The most important co-morbidities for psoriasis are Crohn's disease, inflammatory bowel disease, malignancies, obesity, and cardiovascular diseases, hypertension, and dyslipidaemia.

The treatment of atopic dermatitis and psoriasis has recently seen a significant progress with the introduction of biologics. However, long term control of this condition is yet to be achieved. Often poor management and not so effective treatments significantly increases a patients' risk for developing certain comorbidities, including cancers, severe depression, and cardiovascular diseases such as coronary heart disease, stroke, and heart failure. Long-term atopic dermatitis and psoriasis with frequent relapses and very limited treatment response are jointly a major risk factor for these biggest killers in modern society, with chronic inflammation the common link. This invention addresses the treatment of atopic dermatitis and psoriasis by developing a safe and effective therapeutic option by blocking the expression of genes involved in the pathological processes, thereby preventing skin lesions and disease flare-ups.

While the biologic therapies have revolutionized the treatment of atopic dermatitis and psoriasis, the long-term control of both diseases is still very challenging as these new biologic therapies have significant side-effects limiting their use and still large number of patients are without effective therapeutic option. The main problem with biologics is that they are designed to modulate immune response too broadly. These immunomodulatory therapies may increase the risk of reactivation of latent and chronic infections. In particular, tumour necrosis factor alpha (TNF-α) inhibitors, have been associated with the increased risk of reactivation of tuberculosis (TB) in patients with latent TB, as well as hepatitis B virus (HBV), in patients with chronic HBV infections. Frequently, biologicals have been reported to induce paradoxical psoriasis or palmoplantar pustulosis during the TNF-α treatment. The most common side-effect with biologicals is the upper respiratory tract infection, that can occur even in 30% of patients. Newer biologics, IL-17 inhibitors have shown to induce Crohn's disease and inflammatory bowel disease as severe side effects. But the most common problem with biologics is the increased risk for cancer development. Biologics have been shown to have 3-fold increase in the developing all cancers. However, the data also indicate that intensive treatment with some biologicals have beneficial effect in lowering long term risk for cardiovascular diseases.

Taken together, atopic dermatitis and psoriasis are chronic diseases exhibiting different clinical forms. Their severity varies from mild to moderate and severe, with more severe cases requiring hospitalisation and intensive care. While new biologicals have been developed and they have significantly improved the care of both diseases, very large number of AD and psoriasis patients are still without adequate treatment. In addition, several other chronic skin diseases, like hidradenitis suppurativa and lichen planus, do not have any treatment at all. Chronic systemic inflammation can have different forms on the skin with a systemic effect in increasing the risk for other diseases. Therefore, there is an unmet medical demand for the more specific treatment opportunities with lower frequency of side effects.

The pathogenesis of atopic dermatitis and psoriasis is based on the activation of the inflammatory network that is formed by many different genes. This network works as a self-sustainable system where the individual members support activation of each other. Therefore, once activated, it is difficult to inactivate it, as targeting only single members in the network may not be sufficient. While present new therapeutics for Ad and psoriasis do ameliorate the diseases, their effect is limited and often accompanied by severe side-effects. Therefore, improved alternative treatment options are needed with more complex, more specific and more effective strategies. The present invention describes a therapeutic approach that targets RNA molecules of the activated genes of the inflammatory network that is involved in the atopic dermatitis, psoriasis and other inflammatory conditions.

### DISCLOSURE OF INVENTION

The present invention provides antisense oligonucleotides to inactivate specific gene expression via antisense-based steric hindrance of mRNA translation and to use this technology to treat atopic dermatitis, psoriasis, and chronic inflammatory conditions.

The present invention describes antisense oligonucleotides to sterically block the translation of the genes that cause inflammatory response and have been shown to be involved in atopic dermatitis and psoriasis.

The present invention provides antisense oligonucleotides to reduce the protein synthesis and mRNA translation from the transcripts that are involved in the inflammatory network of atopic dermatitis, psoriasis and chronic inflammation or part thereof.

The present invention provides an isolated or purified antisense oligomer with a modified backbone structure to supress the translation of the mRNA into protein of the selected genes involved in the inflammatory network.

The present invention provides antisense oligonucleotides to enable the steric hindrance that is the mechanism to block the translation of the genes involved in the inflammation.

Preferably the antisense oligomer induces mRNA translational blocking by steric hindrance of the target genes or their transcripts thereof. Preferably the antisense oligomer is selected from the list comprising SEQ ID NOs:1-14.

The present invention provides a method to manipulate the RNA translation for the inflammation inducing genes, the method including the step of:
- providing an isolated or purified antisense oligomer with a modified backbone structure for steric blocking of mRNA translation of the transcripts involved in the inflammatory response, atopic dermatitis and psoriasis or parts thereof and combinations or cocktails thereof and allowing the oligomer(s) to bind to a target nucleic acid site.

The present invention further provides a pharmaceutical, prophylactic, or therapeutic composition to treat, prevent or ameliorate the effects of a disease related to the expression of the inflammatory network gene transcripts in a patient, the composition composing:
- an antisense oligomer with a modified backbone for steric blocking of mRNA translation of the transcripts involved in the inflammatory response, atopic dermatitis, and psoriasis or parts thereof and combinations or cocktails thereof and one or more pharmaceutically acceptable carriers and/or diluents.

A method to treat, prevent or ameliorate the effects of a disease associated with the expression of the genes involved in the inflammatory network of atopic dermatitis, psoriasis and chronic inflammation, comprising the step of:
- administering to the patient an effective amount of an antisense oligomer with a modified backbone for steric blocking of mRNA translation of the transcripts involved in the inflammatory response, atopic dermatitis, and psoriasis or parts thereof and combinations or cocktails thereof, or a pharmaceutical composition comprising the same.

The use of an isolated or purified antisense oligomer with a modified backbone for steric blocking of mRNA translation of the transcripts involved in the inflammatory response, atopic dermatitis, and psoriasis or parts thereof and combinations or cocktails thereof, for the manufacture of a medicament to treat, prevent or ameliorate the effects of a disease associated with atopic dermatitis and psoriasis related gene expression.

A kit to treat, prevent or ameliorate the effects of a disease associated with the expression of the genes involved in the inflammatory network of atopic dermatitis, psoriasis and chronic inflammation in a patient, which kit comprises at least an antisense oligomer with a modified backbone for steric blocking of mRNA translation of the transcripts involved in the inflammatory response, atopic dermatitis, and psoriasis or parts thereof and combinations or cocktails thereof, packages in a suitable container, together with instructions for its use.

Preferably the diseases where genes and transcripts of the inflammatory network and chronic inflammation are involved, are atopic dermatitis, psoriasis, psoriatic arthritis, pustular psoriasis, hidradenitis suppurativa and similar diseases.

These are all potential human diseases and health conditions where the antisense oligomers described in the invention can potentially be used atopic dermatitis, psoriasis, hidradenitis suppurativa, chronic skin inflammations, brain trauma, stroke, brain tissue damage, cardiovascular diseases, myocardial infarction, heart attack.

Our invention is based on the definition and identification of the inflammatory gene network that is specifically activated during inflammatory conditions. The specificity is that certain transcripts of the inflammatory gene network genes are over expressed, and our invention provides, solutions how to downregulate this activation.

### BRIEF DESCRIPTION OF DRAWINGS

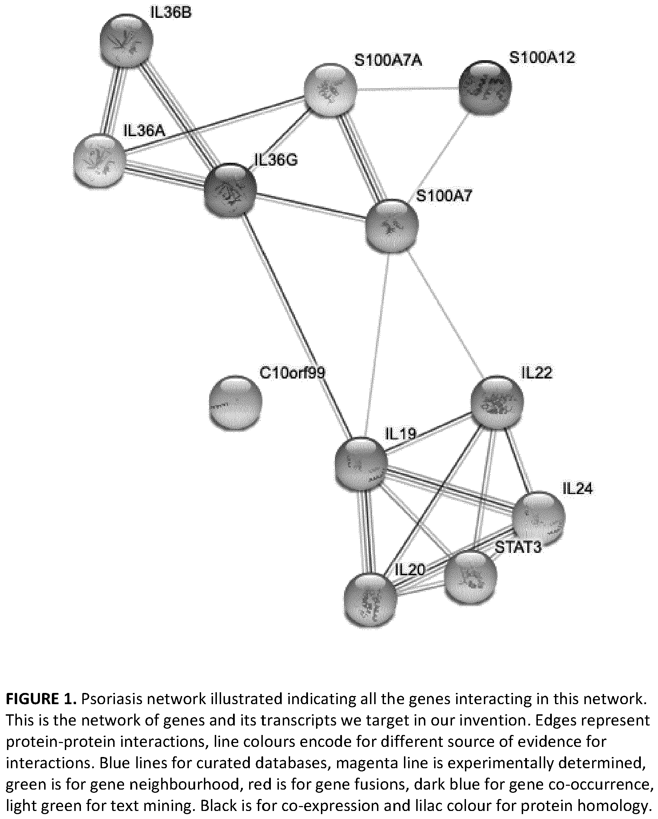

**FIGURE 1****.** Atopic dermatitis and psoriasis network illustrated indicating all the genes interacting in this network. This is the network of genes and its transcripts we target in our invention. Edges represent protein-protein interactions, number of lines encode for different source of evidence for interactions.

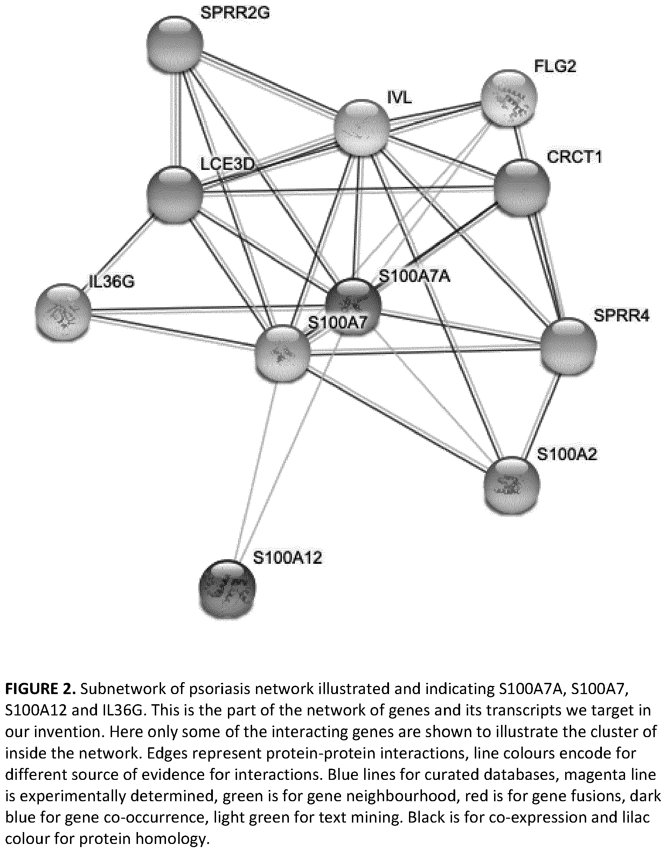

**FIGURE 2****.** Subnetwork of atopic dermatitis and psoriasis network illustrated and indicating S100A7A, S100A7, S100A12 and IL36G. This is the part of the network of genes and its transcripts we target in our invention. Here only some of the interacting genes are shown to illustrate the cluster of inside the network. Edges represent protein-protein interactions, number of lines encode for different source of evidence for interactions.

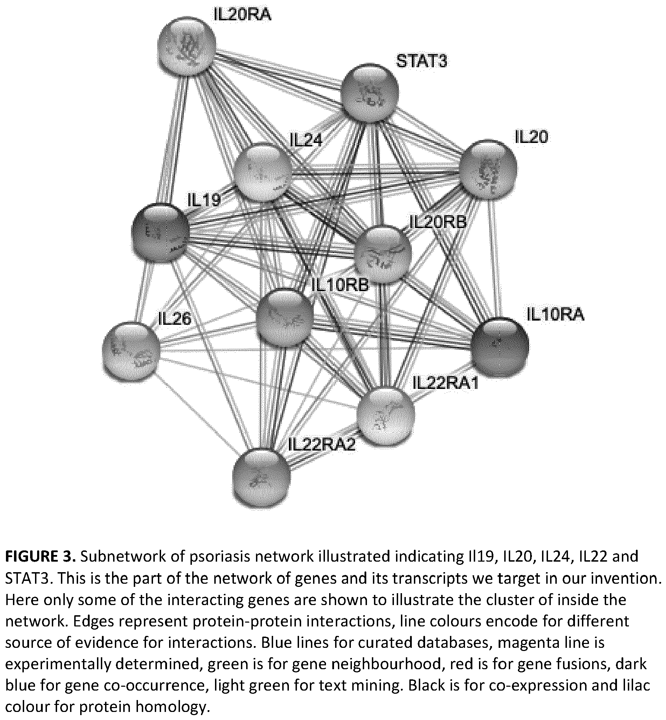

**FIGURE 3****.** Subnetwork of atopic dermatitis and psoriasis network illustrated indicating II19, IL20, IL24, IL22 and STAT3. This is the part of the network of genes and its transcripts we target in our invention. Here only some of the interacting genes are shown to illustrate the cluster of inside the network. Edges represent protein-protein interactions, number of lines encode for different source of evidence for interactions.

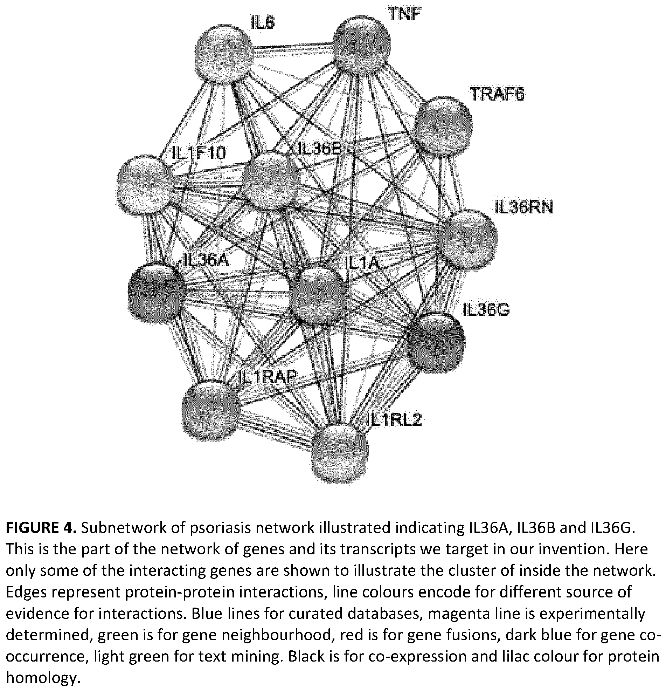

**FIGURE 4****.** Subnetwork of atopic dermatitis and psoriasis network illustrated indicating IL36A, IL36B and IL36G. This is the part of the network of genes and its transcripts we target in our invention. Here only some of the interacting genes are shown to illustrate the cluster of inside the network. Edges represent protein-protein interactions, number of lines encode for different source of evidence for interactions.
**FIGURE 5****.** Translational start AUG site of S100A7 is targeted by the S100A7A_TB1
**FIGURE 6****.** Translational start AUG site of S100A7A is targeted by the S100A7A_TB1
**FIGURE 7****.** The 5'-UTR region of the S100A12-201 transcript of S100A12 gene is targeted by S100A12_TB1.
**FIGURE 8****.** Translational start AUG site of the C10orf99-201 is targeted by C10orf99 _TB1.
**FIGURE 9****.** Translational start AUG site of IL19-204 is targeted by the IL19_TB1.
**FIGURE 10****.** The 5'-UTR region of the IL20-202 transcript of S100A12 gene is targeted by IL20_TB1.
**FIGURE 11****.** Translational start AUG site of IL24-201 is targeted by the IL24_TB1.
**FIGURE 12****.** Translational start AUG site of IL22-202 is targeted by the IL22_TB1.
**FIGURE 13****.** Translational start AUG site of STAT3 is targeted by the STAT3_TB1.
**FIGURE 14****.** The 5'-UTR region of the IL36G-201 transcript of IL36G gene is targeted by IL36G_TB1.
**FIGURE 15****.** The 5'-UTR region of the IL36A-201 transcript of IL36A gene is targeted by IL36A_TB1.
**FIGURE 16****.** Translational start AUG site of IL36B-202 is targeted by the IL36B_TB1.
**FIGURE 17****.** The 5'-UTR region of the GPR15-201 transcript of GPR15 gene is targeted by BOB_TB1.

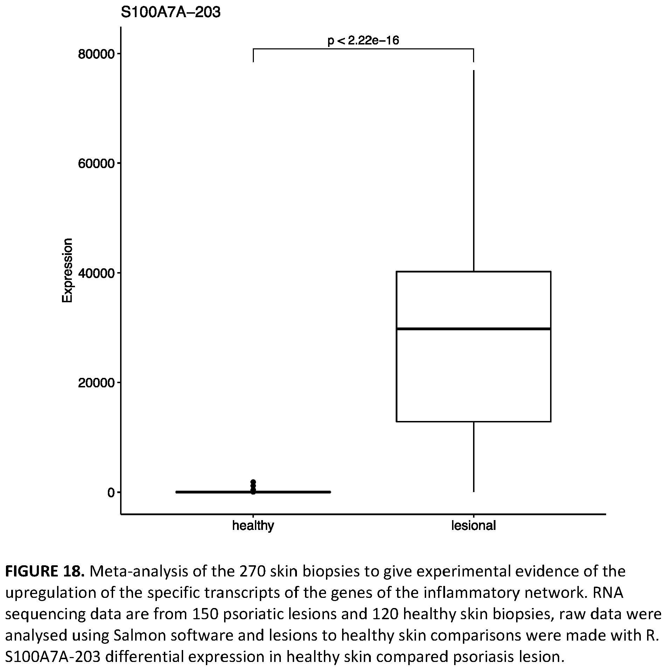

**FIGURE 18****.** Meta-analysis of the 270 skin biopsies to give experimental evidence of the upregulation of the specific transcripts of the genes of the inflammatory network.
RNA sequencing data are from 150 psoriatic lesions and 120 healthy skin biopsies,
raw data were analysed using Salmon software and lesions to healthy skin comparisons were made with R. S100A7A-203 differential expression in healthy skin compared psoriasis lesion.

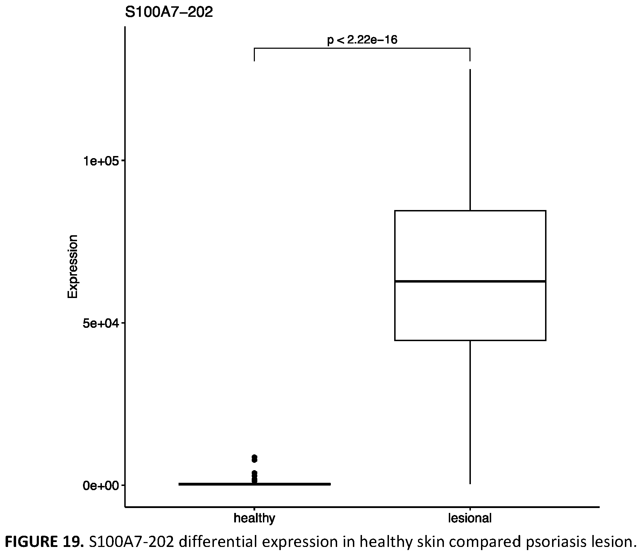

**FIGURE 19****.** S100A7-202 differential expression in healthy skin compared psoriasis lesion.

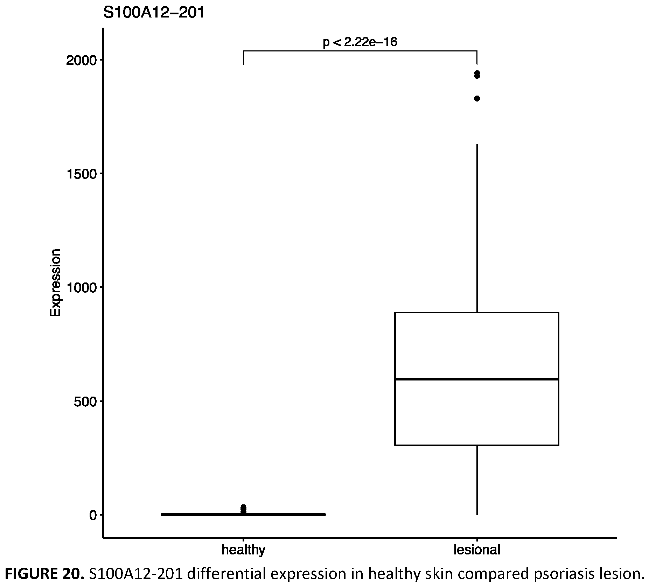

**FIGURE 20****.** S100A12-201 differential expression in healthy skin compared psoriasis lesion.

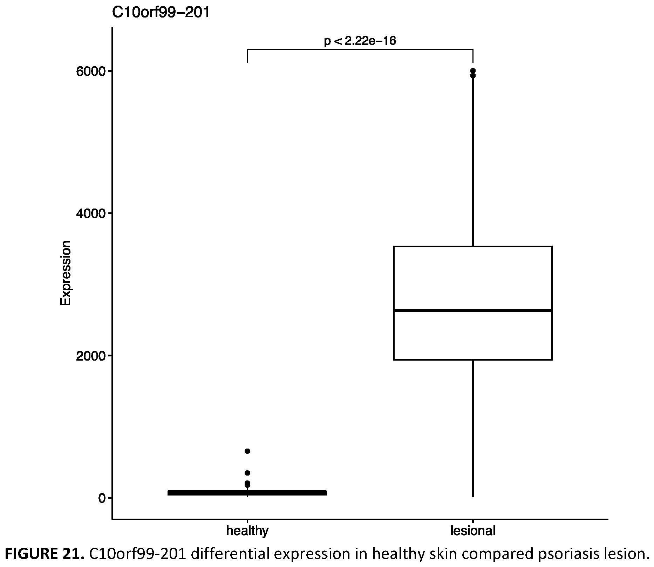

**FIGURE 21****.** C10orf99-201 differential expression in healthy skin compared psoriasis lesion.

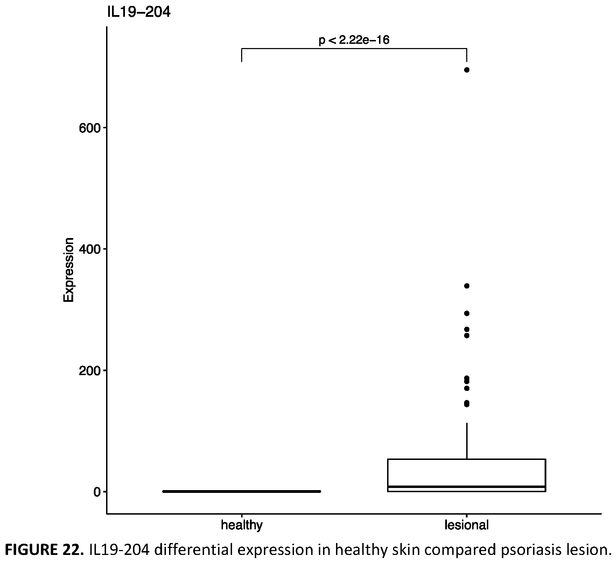

**FIGURE 22****.** IL19-204 differential expression in healthy skin compared psoriasis lesion.

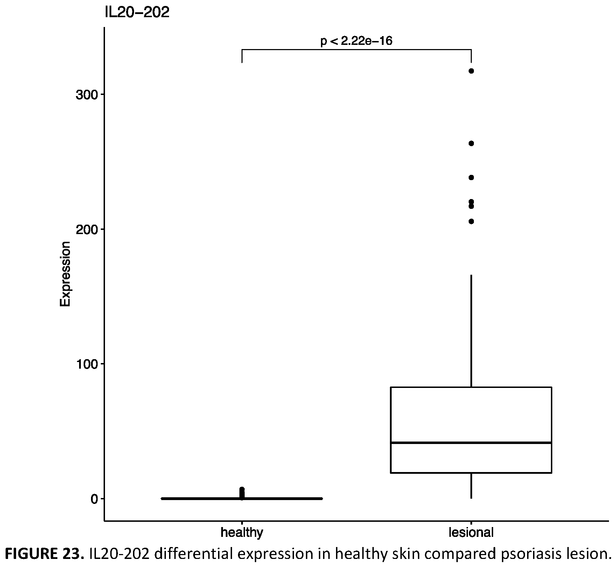

**FIGURE 23****.** IL20-202 differential expression in healthy skin compared psoriasis lesion.

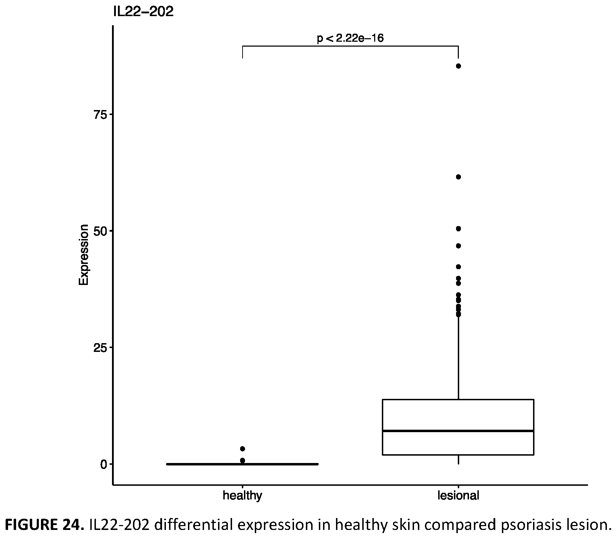

**FIGURE 24****.** IL22-202 differential expression in healthy skin compared psoriasis lesion.

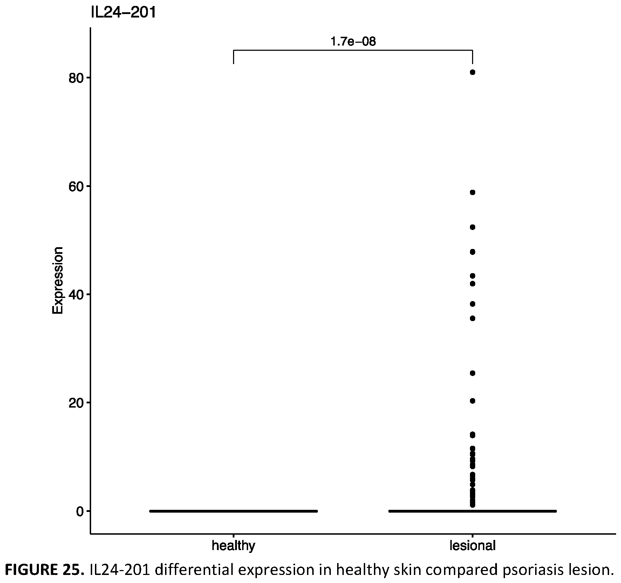

**FIGURE 25****.** IL24-201 differential expression in healthy skin compared psoriasis lesion.

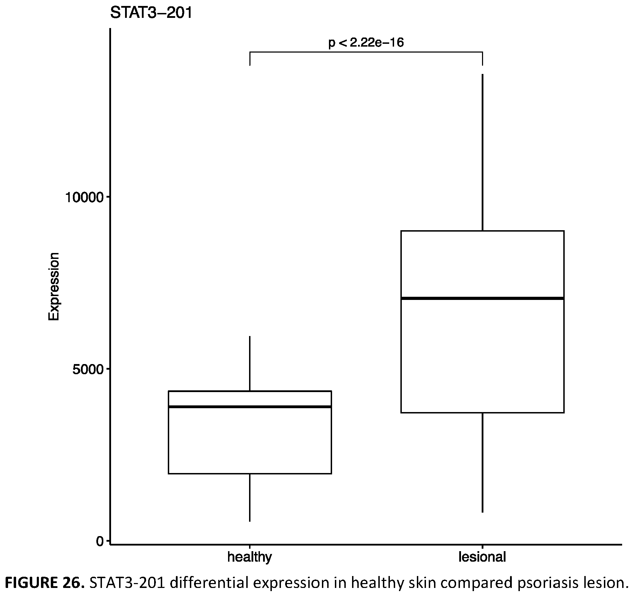

**FIGURE 26****.** STAT3-201 differential expression in healthy skin compared psoriasis lesion.

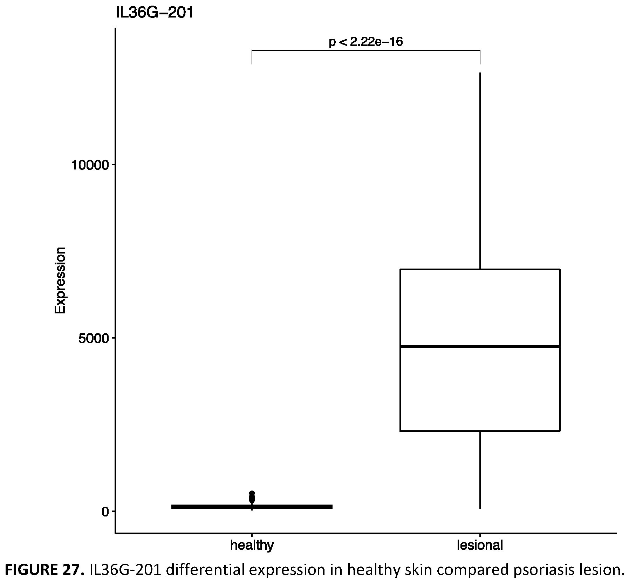

**FIGURE 27****.** IL36G-201 differential expression in healthy skin compared psoriasis lesion.

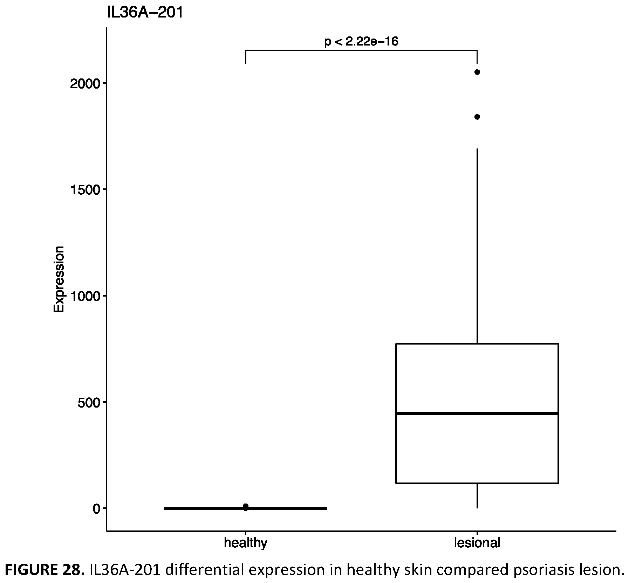

**FIGURE 28****.** IL36A-201 differential expression in healthy skin compared psoriasis lesion.

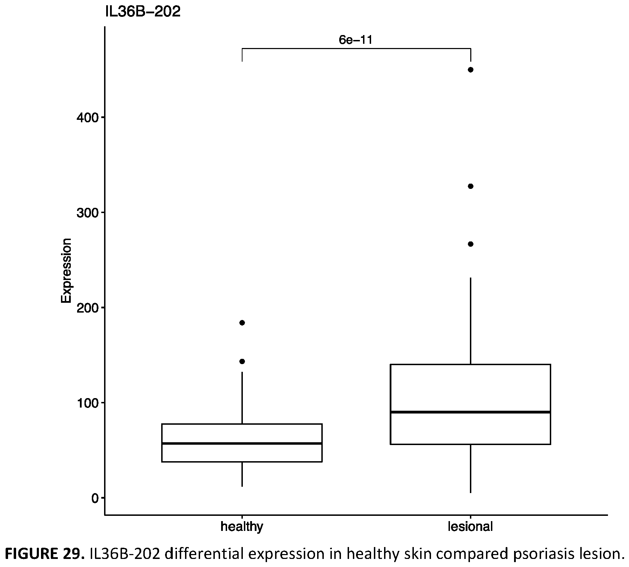

**FIGURE 29****.** IL36B-202 differential expression in healthy skin compared psoriasis lesion.

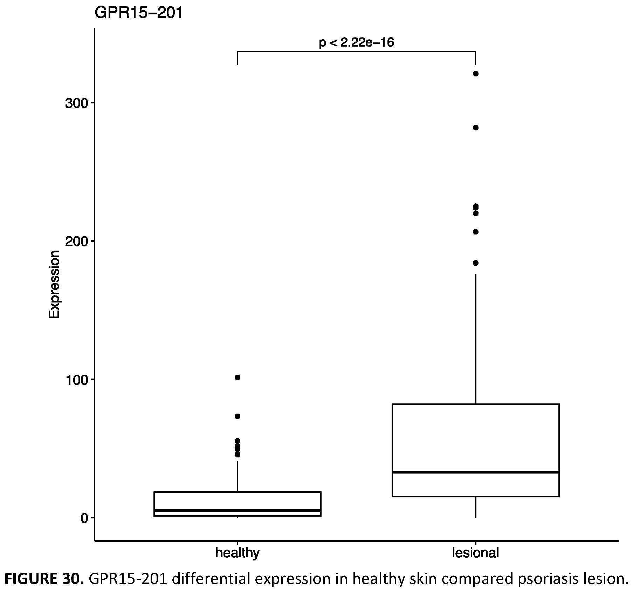

**FIGURE 30****.** GPR15-201 differential expression in healthy skin compared psoriasis lesion.

### DETAILED DESCRIPTION OF INVENTION

### Antisense oligonucleotides

Atopic dermatitis and psoriasis are common chronic inflammatory diseases affecting more than 200 million people worldwide that are caused by the activation of the network of the inflammatory genes and present invention provides a selection of antisense oligonucleotides that block mRNA translation of one or all the 12 members of this inflammatory network. The blocking of mRNA translation blocks the production of proteins from these transcripts.

The present invention is based on the discovery that during inflammatory response the activation of genes is transcript specific, i.e. out of many different existing transcripts, only very specific subtypes are expressed and activated.

In case of some inflammatory genes only one of the multiple transcripts is activated, in some cases multiple transcripts are activated.

The present invention is based on the understanding that the inflammatory network activation is transcript specific and downregulating these specific transcripts would reduce the inflammatory response.

The present invention is based on the understanding that the inflammatory network genes in the context of present invention are S100A7A, S100A7, S100A12, C10orf99, IL19, IL20, IL24, IL22, STAT3, IL36G, IL36A, IL36B and GPR15. The activation of any of these genes causes inflammatory response, the activation is transcript specific and downregulating these specific transcripts will reduce the inflammatory response.

These following transcripts are given as an example to illustrate the specificity of the inflammatory genes' transcripts activation, but is not the exhaustive list:
- Downregulating IL1RL2 specific transcripts like IL1RL2-201, or all of its transcripts leads to the downregulation of inflammatory response;
- Downregulating S100A7A specific transcripts like S100A7A-203, or all of its transcripts leads to the downregulation of inflammatory response;
- Downregulating S100A7 specific transcripts like S100A7-202, or all of its transcripts leads to the downregulation of inflammatory response;
- Downregulating S100A12 specific transcripts like S100A12-201, or all of its transcripts leads to the downregulation of inflammatory response;
- Downregulating C10orf99 specific transcripts like C10orf99-201, or all of its transcripts leads to the downregulation of inflammatory response;
- Downregulating IL19 specific transcripts like IL19-204, or all of its transcripts leads to the downregulation of inflammatory response;
- Downregulating IL20 specific transcripts like IL20-202, or all of its transcripts leads to the downregulation of inflammatory response;
- Downregulating IL24 specific transcripts like IL24-201, or all of its transcripts leads to the downregulation of inflammatory response;
- Downregulating IL22 specific transcripts like IL22-202, or all of its transcripts leads to the downregulation of inflammatory response;
- Downregulating STAT3 specific transcripts like STAT3-201, or all of its transcripts leads to the downregulation of inflammatory response;
- Downregulating IL36G specific transcripts like IL36G-201, or all of its transcripts leads to the downregulation of inflammatory response;
- Downregulating IL36A specific transcripts like IL36A-201, or all of its transcripts leads to the downregulation of inflammatory response;
- Downregulating IL36B specific transcripts like IL36B-202, or all of its transcripts leads to the downregulation of inflammatory response;
- Downregulating GPR15 specific transcripts like GPR15-201, or all of its transcripts leads to the downregulation of inflammatory response;

These examples show only one possible approach how to block transcript specific translation, to downregulate gene or genes in the inflammatory network and to reduce psoriasis or other inflammatory diseases.

Atopic dermatitis and psoriasis are common chronic inflammatory diseases that are caused by the activation of the network of the inflammatory genes. This gene activation is a self -perpetuating process where each of the network member stimulates the activation of other members. The activation of these inflammatory network genes is multistage process involving the synthesis of mRNA and translation of the mRNA into bioactive proteins know by their specific functional names like cytokines, chemokines, alarmins etc. Inflammatory response is an innate immune response, and it is designed to protect the organism from intrudes and this system recognises the foreign molecules. However, in case of atopic dermatitis and psoriasis and other inflammatory diseases, this protective response has become too intensive, out of the control and self-perpetuating. That out-of-control defensive response leads to the tissue damage and tissue reconstruction and remodelling what are typical chronic inflammatory changes in the tissues. These changes in the tissue remodelling can lead to permanent changes and even to the cancer.

The inflammatory response is a protective response, and it is very tightly regulated by multiple different genes to form a dense interacting network. Many different genes interact in it to provide strong and defensive response against the foreign materials, infection, pathogen or against trauma or cellular damage or dying cells. This inflammatory response network is initiated by the damage-associated molecular patterns (DAMPs) or danger signals or alarm ins and is a part of innate response. This invention is related to the very specific parts of the inflammatory network that is activated during the psoriasis or during any other skin inflammation or any tissue damage. Our invention targets specifically some of the S100 proteins and interleukins that are released in response to the tissue injury and cause inflammation.

The present invention provides antisense oligomers to block mRNA translation of several members of the inflammatory network. These antisense oligomers can be used alone or in combination and the precise combination will depend on the pathological process it is used against and personal individual characteristics like genetic variations.

The present invention does not induce increased degradation of RNA via recruitment of RNAse H. The antisense oligomers in the present invention bind to the complementary RNA and knock down the gene expression via an RNAse H-independent steric blocking mechanism. Their action is based on the high mRNA binding affinity of morpholinos.

The antisense oligomers we propose here can be morpholino oligomers, phosphorodiamidate (PMO) or thiophosphoramidate (TMO) morpholino oligomers or any other morpholino modifications. Morpholino oligomers have exquisite specificity and very high affinity making them highly reliable and predictable in their action. Morpholino oligomers are very stable in the cells, they are uncut by nucleases.

The proposed antisense oligonucleotides will block mRNA translation into the proposed signalling proteins, and this leads to the downregulation of the inflammatory activity. Translational blocking is achieved by the steric hindrance that stops the translational initiation at the 5'UTR of the mRNA.

The antisense oligomers presented in this invention will stop the inflammatory response and will restore the normal functioning of the tissue by blocking the translation of the mRNA of the genes involved in the inflammatory response. Stopping inflammatory response is important to avoid long term damage and functional deficiency of the tissue, therefore the antisense oligonucleotides will stop the tissue destruction and help to restore the damaged tissue. The proposed antisense oligonucleotides can avoid tissue damage caused by the long-term inflammation and long-term protective response induced by the DAMPs.

The proposed antisense oligonucleotides can be used for any kind of causes when the inflammatory response is too strong and out of the control. This can happen after large tissue damage, like myocardial infarction or stroke in the brain. These antisense oligonucleotides can be used to reduce the damage of the myocardial muscle after heart attack and infraction.

The proposed antisense oligonucleotides can also protect cell from the damage of the stroke in the brain and can be used to treat stroke damage at any stages of the stroke.

The proposed antisense oligonucleotides will restore the functioning of the cells after slowly progressing chronic damages caused by the protein aggregations, and against the slowly progressing damage caused by the protein aggregation.

The first aspect of the invention provides the antisense oligomer sequences that are capable of binding to a selected targets in the inflammatory network and block their mRNA translation. An isolated or purified antisense oligomers are provided for steric hindrance and translational blocking of the selected mRNAs.

The "isolated" means a material that is purified substantially or essentially is free from components that normally accompany it in its native state. "Isolated polynucleotide" or "isolated oligonucleotide", as used herein, may refer to a polynucleotide that has been purified or removed from the sequences that are adjacent in the genome. The term "isolating" as it relates to cells refers to the purification of cells from a source subject (subject with a disease). In the context of mRNA or protein, "isolating" refers to the recovery of mRNA or protein from a source (e.g. cells).

An antisense oligomer can be said to be "directed to" or "targeted against" a target sequence with which it hybridizes. In certain embodiments, the target sequence includes a region from the 5'cap to about 25 bases 3' to the AUG translational start site in the post-spliced mRNA.

In certain embodiments, the antisense oligomer has sufficient sequence complementarity to a target mRNA (i.e., the mRNA for which translation is blocked) to block a region of a target mRNA (e.g., translational start site AUG) in an effective manner. In exemplary embodiments, such blocking mRNA of atopic dermatitis and psoriasis network genes serves to block translation by binding specifically the sequence of RNA and causing steric hindrance.

An antisense oligomer having a sufficient sequence complementarity to a target mRNA sequence to block translation of the target mRNA means that the antisense oligomer has a sequence sufficient to bind with high affinity the sequence that lies within the region from the 5' cap through the first 25-bases of coding sequence to invade RNA secondary structure and to trigger the steric hindrance or steric blocking the translation of the targeted RNA.

Selected antisense oligomers can be made shorter, e.g., about 12 bases, or longer, e.g., about 50 bases, and include a small number of mismatches, as long as the sequence is sufficiently complementary to effect translational blocking upon hybridization to the target sequence.

Preferably, the antisense oligomer is selected from the group comprising the sequences set forth in Table 1. Preferably, the antisense oligomer is selected from the group comprising the sequences in SEQ ID NOs: 1 -14, more preferably SEQ ID NOs: 1-3, 4-7, 9 and 14, even more preferably SEQ ID NOs: 1-3, 9 and 14.

In certain embodiments, the degree of complementarity between the target sequence and antisense oligomer is sufficient to form a stable duplex. The region of complementarity of the antisense oligomers with the target RNA sequence may be as short as 8-11 bases, but can be 12-15 bases or more, e.g., 10-50 bases, including all integers in between these ranges. An antisense oligomer of about 16-17 bases is generally long enough to have a unique complementary sequence. In certain embodiments, a minimum length of complementary bases may be required to achieve the requisite binding Tm, as discussed herein.

In certain embodiments, oligomers as long as 50 bases may be suitable, where at least a minimum number of bases, e.g., 10-12 bases, are complementary to the target sequence. In general, however, facilitated, or active uptake in cells is optimized at oligomer lengths of less than about 30 bases. For phosphorodiamidate morpholino oligomer (PMO) antisense oligomers, an optimum balance of binding stability and uptake generally occurs at lengths of 18-35 bases. Included are antisense oligomers (e.g., Vivo-Morpholinos, CPP-PMOs, PPMOs, PMOs, PMO-X, PMO+ PNAs, LNAs, 2'-OMe, 2'MOE) that consist of anything between 10-50 bases.

In certain embodiments, antisense oligomers may be 100% complementary to the target sequence, or may include mismatches, e.g., to accommodate variants, as long as a heteroduplex formed between the oligonucleotide and target sequence is sufficiently stable to withstand the action of cellular nucleases and other modes of degradation that may occur in vivo. Hence, certain oligonucleotides may have about or at least about 70% sequence complementarity, e.g., 70% - 100% sequence complementarity, between the oligonucleotide and the target sequence.

Mismatches, if present, are typically less destabilizing toward the end regions of the hybrid duplex than in the middle. The number of mismatches allowed will depend on the length of the oligonucleotide, the percentage of G:C base pairs in the duplex, and the position of the mismatch(es) in the duplex, according to well understood principles of duplex stability. Although such an antisense oligomer is not necessarily 100% complementary to the target sequence, it is effective to bind to the target sequence stably and specifically, such that translation of the target mRNA can be blocked.

The stability of the duplex formed between an antisense oligomer and a target sequence is a function of the binding Tm and the susceptibility of the duplex to cellular enzymatic cleavage. The Tm of an oligonucleotide with respect to complementary-sequence RNA may be measured by conventional methods. In certain embodiments, antisense oligomers may have a binding Tm, with respect to a complementary-sequence RNA, of greater than body temperature and preferably greater than about 45°C or 50°C. Tm's in the range 60-90°C or even 90-110°C are also included. For the PMO translational blocking the typical Tm is in the range 90-110°C.

Additional examples of variants include antisense oligomers having about or at least about 70% sequence identity or homology, e.g., 70% - 100% sequence identity or homology, over the entire length of any of in SEQ ID NOs: 1 -14, more preferably SEQ ID NOs: 1-3, 4-7, 9 and 14, even more preferably SEQ ID NOs: 1-3, 9 and 14, or the sequences provided in Table 1.

More specifically, there is provided an antisense oligomer capable of binding to a selected target site to block translation of the mRNA transcript in of the inflammatory network genes shown in the Table 1 or part thereof. The antisense oligomer is preferably selected from those provided in Table 1 or in SEQ ID NOs: 1 -14, more preferably SEQ ID NOs: 1-3, 4-7, 9 and 14, even more preferably SEQ ID NOs: 1-3, 9 and 14.

The steric blocking of mRNA translation preferably induces hindrance or the block of translation within the region from the 5' cap through the first 25-bases of coding sequence. No functional protein will be translated from the targeted RNAs as result of effective steric hindrance. Usually, the target sequence for translational blocking is in the post-spliced mRNA in the region from the 5'cap to about 25 bases 3' to the AUG translational start site.

The antisense oligomers of the invention may be a combination of two or more antisense oligomers capable of binding to a selected target to induce translational blocking in any gene of the inflammatory network gene. The combination may be a cocktail of two or more antisense oligomers and/or a construct comprising two or more or two or more antisense oligomers joined together.

The antisense oligomer may comprise a sequence designed to target only 5'-UTR or only AUG region or AUG can be in the centre of the antisense. Preferably such antisense oligomers with sequence directed to target any region from the 5'-UTR cap through the first 25-bases of translational start site are phosphorodiamidate morpholino oligomers (PMOs, morpholinos), vivo-morpholinos or thiophosphoramidate morpholino oligomers (TMOs).

**Table 1: List of antisense oligonucleotide sequences used in this patent**

| **Seq ID** | **AO nomenclature** | **Sequence 5' ->** 3' | **Genomic position, GRCh38** | **Length (bp)** |
|---|---|---|---|---|
| 1 | S100A7A_TB1 (-8+17) | GCTTGAGTGTTGCTCATCTTTGCTT | 1:153458997-153459021 | 25 |
| 2 | S100A12_TB1 (-43-19) | ACCCCTCAATGCACAGGAATGTGGA | 1:153375550-153375574 | 25 |
| 3 | C10orf99_TB1 (-17+8) | AGCCTCATGGTGAGAAGGCAGATTT | 10:84173835-84173859 | 25 |
| 4 | IL19_TB1 (-8+17) | ACACACTGTAACTTCATGCCCCGTG | 1:206799001 -206799006;206836660-206836679 | 25 |
| 5 | IL20_TB1 (-84-60) | ACTGAGTTTCCCCTCCTCCAAGTGT | 1:206865769-206865793 | 25 |
| 6 | IL24_TB1 (-19+6) | ATTCATCTCTCAGTCTCGTGTTCCT | 1:206897814-206897838 | 25 |
| 7 | IL22_TB1 (-13+12) | CAGGGCGGCCATTGCAGACAATTCT | 12:68253437-68253461 | 25 |
| 8 | STAT3_TB1 (-5+20) | AGCTGATTCCATTGGGCCATCCTGC | 17:42348497-42348521 | 25 |
| 9 | IL36G_TB1 (-79-55) | ACTGAATCGTGGCTCCAGCAGCTTC | 2:112978019-112978043 | 25 |
| 10 | IL36A_TB1 (-61-37) | GTCCTTTTATGTGCAGACCGACCCC | 2:113005811-113005835 | 25 |
| 11 | IL36B_TB1 (-16+9) | TGGGTTCATGATGTCTTCAGAGCCT | 2:113031701-113031725 | 25 |
| 12 | IL1RL2_TB1 (+1+25) | ACCCGCAGAGCAGCAAGGACCACAT | 2:102187868-102187892 | 25 |
| 13 | IL1RL2_TB2 (-12+13) | CGAGCCCTGTCATGCCACTTCCATG | 2:102187003-102187027 | 25 |
| 14 | BOB_TB1 (-33-9) | AGCAGATGCCAAATCTGGTGAGTTT | 3:98532001-98532025 | 25 |

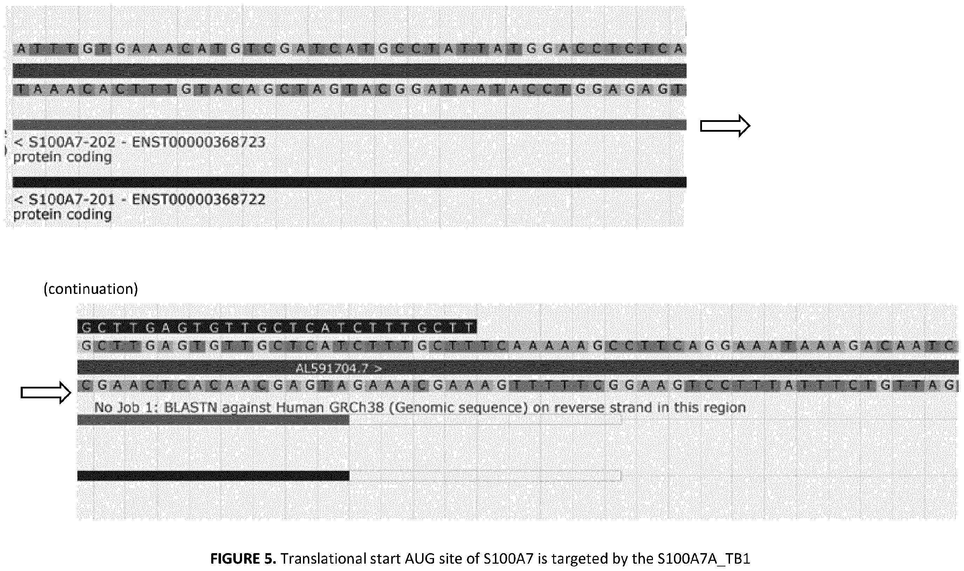

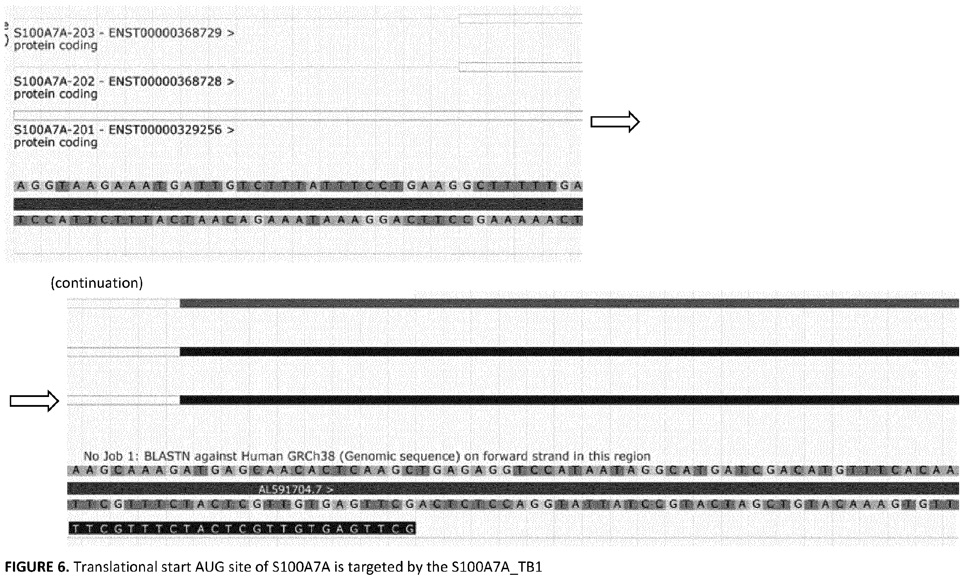

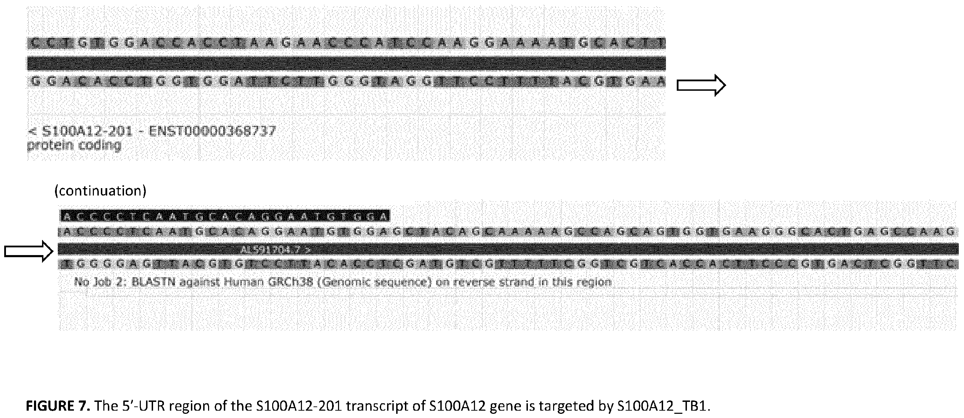

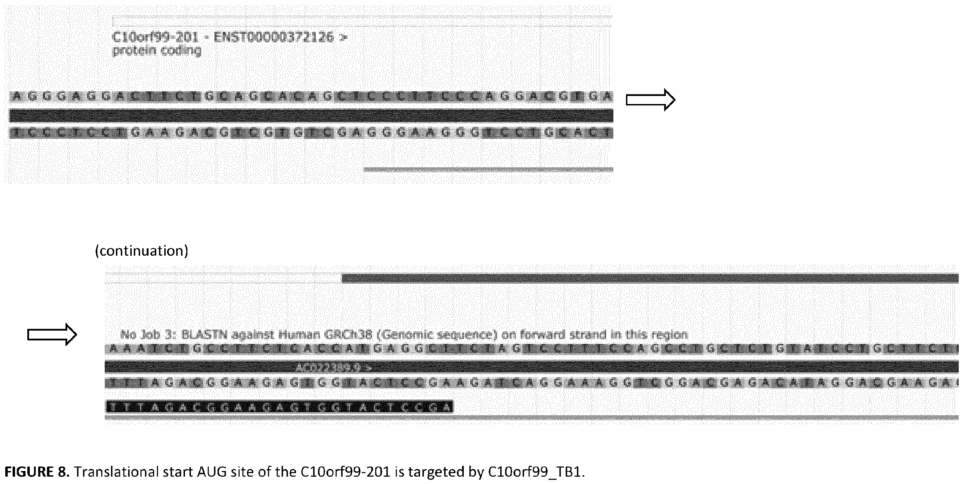

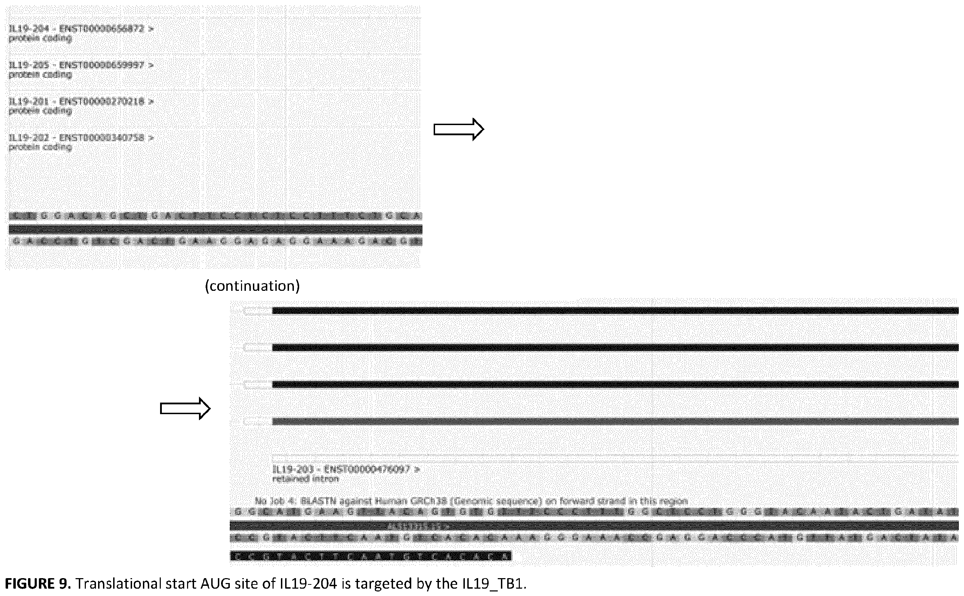

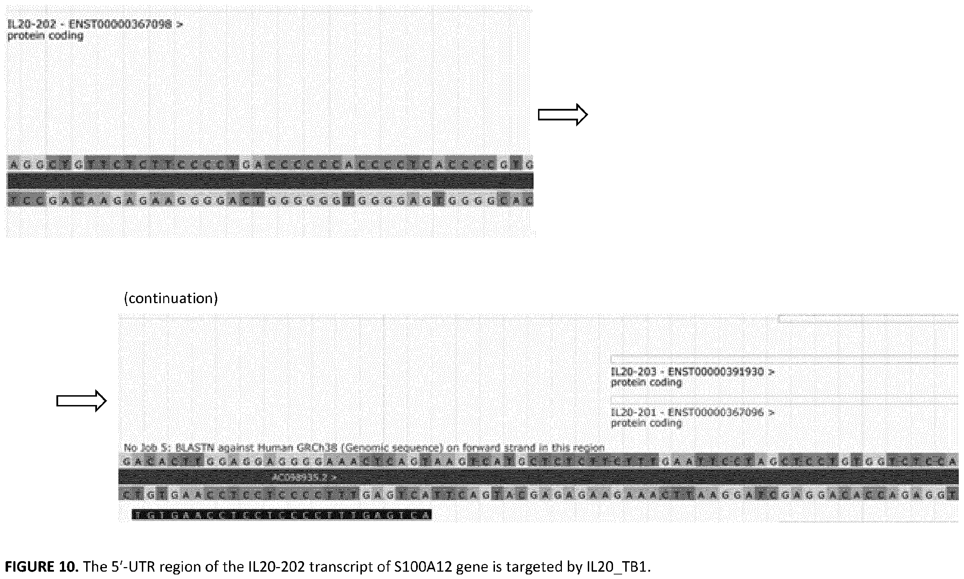

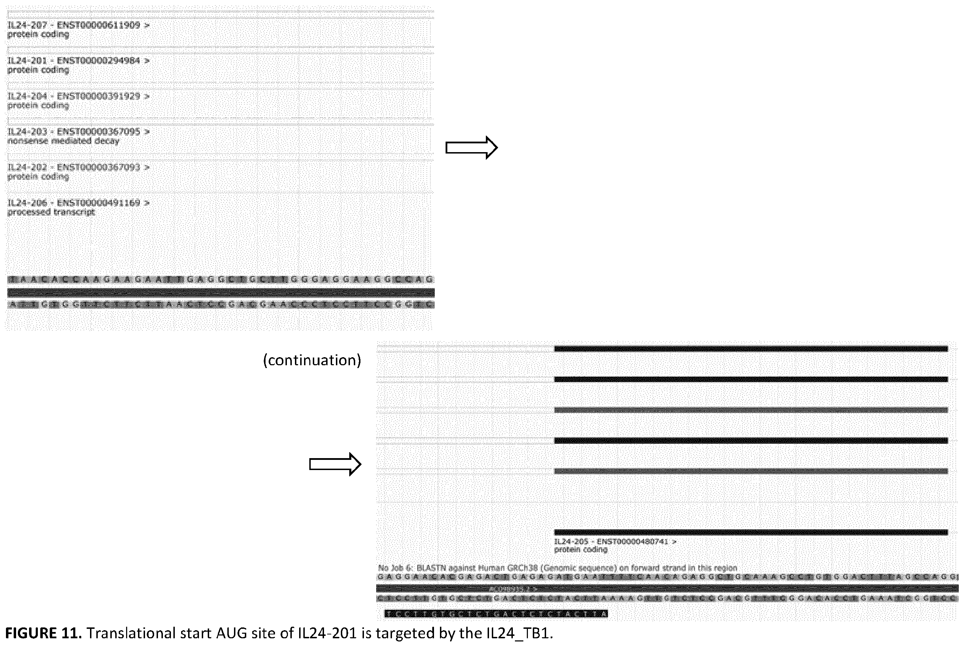

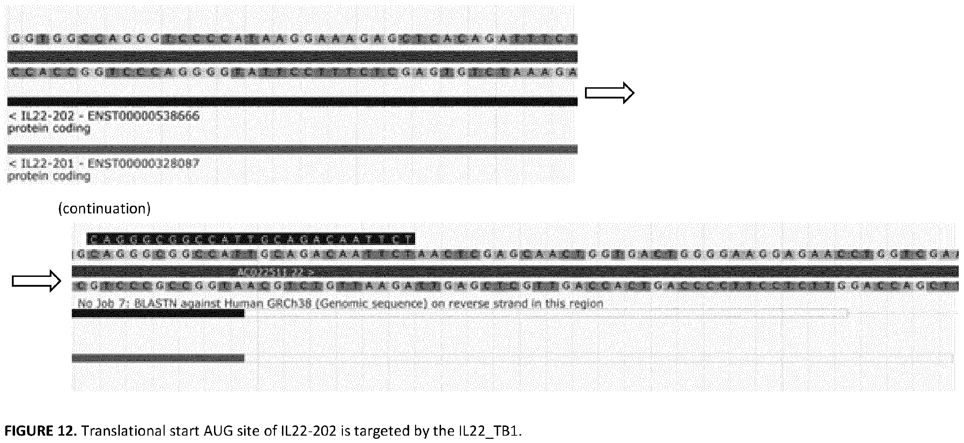

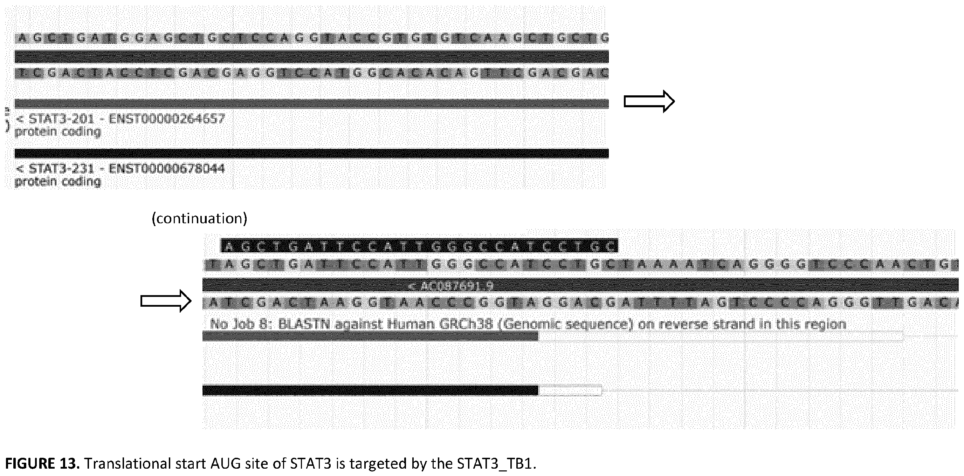

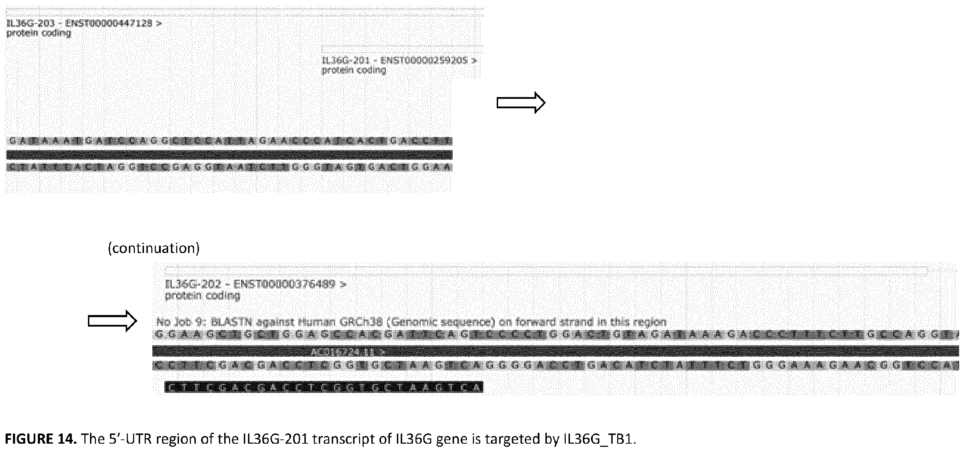

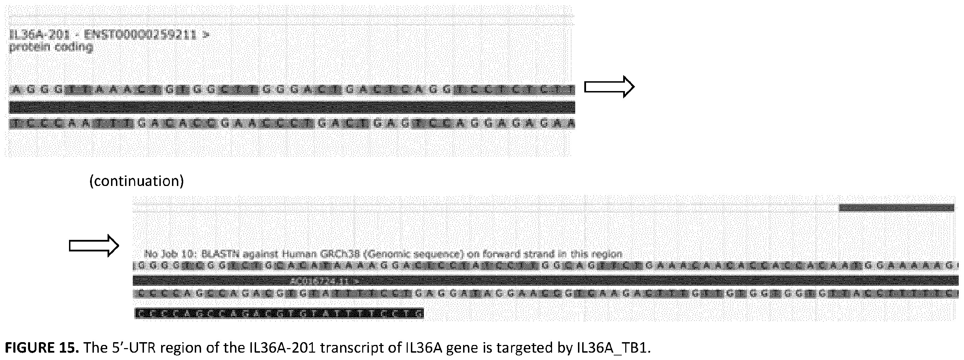

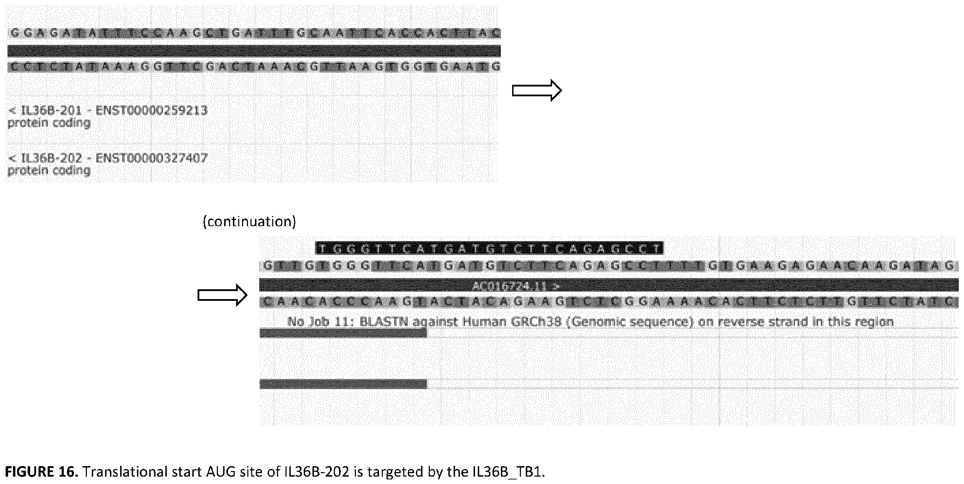

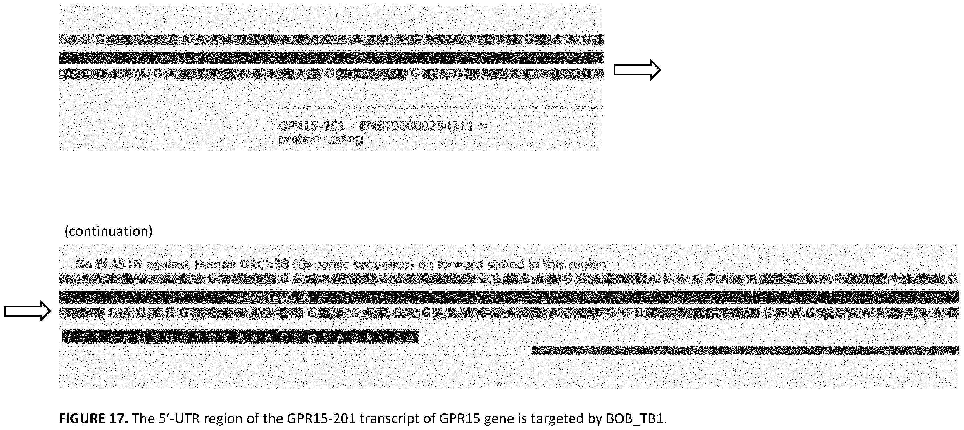

Sequences 1-14 were aligned and indicated in the FIGURES 5 to 17. FIGURES 5-17 show the targeting regions of the antisense oligomers to induce translational blocking of the inflammatory network genes.

There is also provided a method for blocking translation in the inflammatory network gene transcripts, the method including the step of:
a) providing one or more of the antisense oligomers as described herein and allowing the oligomer(s) to bind to a target nucleic acid site.

According to yet another aspect of the invention, there is provided blocking translation from target nucleic acid sequence in the inflammatory network gene transcripts comprising the DNA equivalents of the nucleic acid sequences selected from Table 1 or the group consisting of in SEQ ID NOs: 1 -14, more preferably SEQ ID NOs: 1-3, 4-7, 9 and 14, even more preferably SEQ ID NOs: 1-3, 9 and 14, and sequences complementary thereto.

Designing antisense oligomers to mask the region from the 5'-UTR cap through the first 25-bases of translational start site may not necessarily generate translational blocking. Furthermore, the inventors have discovered that size or length of the antisense oligomer itself is not always a primary factor when designing antisense oligomers. With some targets antisense oligomers as short as 20 bases were able to induce some translational blocking, in certain cases more efficiently than other longer oligomers directed to the same region.

The inventors have also discovered that there does not appear to be any standard motif that can be blocked or masked by antisense oligomers to block translation. It has been found that antisense oligomers must be designed, and their individual efficacy evaluated empirically.

More specifically, the antisense oligomer may be selected from those set forth in Table 1. The sequences are preferably selected from the group consisting of any one or more of any one or in SEQ ID NOs: 1-14, more preferably SEQ ID NOs: 1-3, 4-7, 9 and 14, even more preferably SEQ ID NOs: 1-3, 9 and 14, and combinations or cocktails thereof. This includes sequences that can hybridise to such sequences under stringent hybridisation conditions, sequences complementary thereto, sequences containing modified bases, modified backbones, and functional truncations or extensions thereof which possess or modulate mRNA translation in the inflammatory network gene transcripts.

The oligomer and the DNA, cDNA or RNA are complementary to each other when enough corresponding positions in each molecule are occupied by nucleotides that can hydrogen bond with each other. Thus, "specifically hybridisable" and "complementary" are terms that are used to indicate a sufficient degree of complementarity or pairing such that stable and specific binding occurs between the oligomer and the DNA, cDNA or RNA target. It is understood in the art that the sequence of an antisense oligomer need not be 100% complementary to that of its target sequence to be specifically hybridisable. An antisense oligomer is specifically hybridisable when binding of the compound to the target DNA or RNA molecule interferes with the normal function of the target DNA or RNA product, and there is a sufficient degree of complementarity to avoid non-specific binding of the antisense oligomer to non-target sequences under conditions in which specific binding is desired, i.e., under physiological conditions in the case of in vivo assays or therapeutic treatment, and in the case of in vitro assays, under conditions in which the assays are performed.

Selective hybridisation may be under low, moderate, or high stringency conditions, but is preferably under high stringency. Those skilled in the art will recognise that the stringency of hybridisation will be affected by such conditions as salt concentration, temperature, or organic solvents, in addition to the base composition, length of the complementary strands and the number of nucleotide base mismatches between the hybridising nucleic acids. Stringent temperature conditions will generally include temperatures more than 30°C, typically more than 37°C, and preferably more than 45°C, preferably at least 50°C, and typically 60°C-110°C. Stringent salt conditions will ordinarily be less than 1000 mM, typically less than 500 mM, and preferably less than 200 mM. However, the combination of parameters is much more important than the measure of any single parameter. An example of stringent hybridisation conditions is 65°C and 0.1 x SSC (1 x SSC = 0.15 M NaCl, 0.015 M sodium citrate pH 7.0). Thus, the antisense oligomers of the present invention may include oligomers that selectively hybridise to the sequences provided in Table 1, or in SEQ ID NOs: 1-14, more preferably SEQ ID NOs: 1-3, 4-7, 9 and 14, even more preferably SEQ ID NOs: 1-3, 9 and 14.

It will be appreciated that the codon arrangements that under certain circumstances, a plurality of antisense oligomers may need to be selected by the method of the invention wherein each is directed to a different region responsible for inducing translational blocking. At a given ionic strength and pH, the Tm is the temperature at which 50% of a target sequence hybridizes to a complementary polynucleotide. Such hybridization may occur with "near" or "substantial" complementarity of the antisense oligomer to the target sequence, as well as with exact complementarity.

Typically, selective hybridisation will occur when there is at least about 55% identity over a stretch of at least about 14 nucleotides, preferably at least about 65%, more preferably at least about 75% and most preferably at least about 95% identity with the nucleotides of the antisense oligomer. The length of homology comparison, as described, may be over longer stretches and in certain embodiments will often be over a stretch of at least about nine nucleotides, usually at least about 12 nucleotides, more usually at least about 20, often at least about 21, 22, 23 or 24 nucleotides, at least about 25, 26, 27 or 28 nucleotides, at least about 29, 30, 31 or 32 nucleotides, at least about 36 or more nucleotides.

Thus, the antisense oligomer sequences of the invention preferably have at least 75%, more preferably at least 85%, more preferably at least 90% homology to the sequences shown in the sequence listings herein. More preferably there is at least 91%, more preferably at least 96% homology. Generally, the shorter the length of the antisense oligomer, the greater the homology required to obtain selective hybridisation. Consequently, where an antisense oligomer of the invention consists of less than about 30 nucleotides, it is preferred that the percentage identity is greater than 75%, preferably greater than 85% compared with the antisense oligomers set out in the sequence listings herein. Nucleotide homology comparisons may be conducted by sequence comparison programs such as the GCG Wisconsin Bestfit program or GAP (Deveraux et al., 1984, Nucleic Acids Research 12, 387-395). In this way sequences of a similar or substantially different length to those cited herein could be compared by insertion of gaps into the alignment, such gaps being determined, for example, by the comparison algorithm used by GAP.

The antisense oligomers of the present invention may have regions of reduced homology, and regions of exact homology with the target sequence. It is not necessary for an oligomer to have exact homology for its entire length. For example, the oligomer may have continuous stretches of at least 4 or 5 bases that are identical to the target sequence, preferably continuous stretches of at least 6 or 7 bases that are identical to the target sequence, more preferably continuous stretches of at least 8 or 9 bases that are identical to the target sequence. The oligomer may have stretches of at least 10 to 26 bases that are identical to the target sequence. The remaining stretches of oligomer sequence may be intermittently identical with the target sequence; for example, the remaining sequence may have an identical base, followed by a non-identical base, followed by an identical base.

The term "modulate" or "modulates" includes to "increase" or "decrease" one or more quantifiable parameters, optionally by a defined and/or statistically significant amount. The terms "increase" or "increasing," "enhance" or "enhancing," or "stimulate" or "stimulating" refer generally to the ability of one or antisense oligomers or compositions to produce or cause a greater physiological response (i.e., downstream effects) in a cell or a subject relative to the response caused by either no antisense oligomer or a control compound. The terms "decreasing" or "decrease" refer generally to the ability of one or more antisense oligomers or compositions to produce or cause a reduced physiological response (i.e., downstream effects) in a cell or a subject relative to the response caused by either no antisense oligomer or a control compound.

Relevant physiological or cellular responses (*in vivo* or *in vitro*) will be apparent to persons skilled in the art and may include decrease in the translation from the mRNA, decrease in the amount of inflammatory network genes coding mRNA or decrease in the expression of functional inflammatory network gene proteins in a cell, tissue, or subject in need thereof.

An "decreased" or "reduced", or increased amount is typically a statistically significant amount, and may include a decrease or increase that is 1.1, 1.2, 2-50 or more times (e.g., 500, 1000 times) (including all integers and decimal points in between and above 1) more or less than the amount produced when no antisense oligomer is present (the absence of an agent) or a control compound is used.

The term "reduce" or "inhibit" may relate generally to the ability of one or more antisense oligomers or compositions to "decrease" a relevant physiological or cellular response, such as a symptom of a disease or condition described herein, as measured according to routine techniques in the diagnostic art. Relevant physiological or cellular responses (in vivo or in vitro) will be apparent to persons skilled in the art, and may include reductions in the symptoms or pathology of a disease such as psoriasis or chronic inflammatory diseases.

A "decrease" in a response may be statistically significant as compared to the response produced by no antisense oligomer or a control composition, and may include a 1% - 100% decrease, including all integers in between.

The length of an antisense oligomer may vary, if it is capable of binding selectively to the intended location within the mRNA molecule. The length of such sequences can be determined in accordance with selection procedures described herein. Generally, the antisense oligomer will be from about 10 nucleotides in length, up to about 50 nucleotides in length. It will be appreciated, however, that any length of nucleotides within this range may be used in the method. Preferably, the length of the antisense oligomer is between 10 to 40 nucleotides in length or 20 to 30 nucleotides in length, most preferably about 25 to 30 nucleotides in length. For example, the oligomer may be 20 - 25 nucleotides in length.

As used herein, an "antisense oligomer" refers to a linear sequence of nucleotides, or nucleotide analogs, that allows the nucleobase to hybridize to a target sequence in an RNA by Watson-Crick base pairing, to form an oligomer: RNA heteroduplex within the target sequence. The terms "antisense oligomer", "antisense oligonucleotide", "oligomer" and "antisense compound" may be used interchangeably to refer to an oligonucleotide or oligonucleotide analogue. The cyclic subunits may be based on ribose or another pentose sugar or, in certain embodiments, a morpholino group (see description of morpholino oligonucleotides below). Also contemplated are peptide nucleic acids (PNAs), locked nucleic acids (LNAs), 2'-O-methyl and 2'-O-methoxyethyl oligonucleotides, among other antisense agents known in the art.

Included are non-naturally occurring antisense oligomers, or "oligonucleotide analogs", including antisense oligomers or oligonucleotides having (i) a modified backbone structure, e.g., a backbone other than the standard phosphodiester linkage found in naturally occurring oligo-and polynucleotides, and/or (ii) modified sugar moieties, e.g., morpholino moieties rather than ribose or deoxyribose moieties. Oligonucleotide analogs support bases capable of hydrogen bonding by Watson-Crick base pairing to standard polynucleotide bases, where the analog backbone presents the bases in a manner to permit such hydrogen bonding in a sequence-specific fashion between the oligonucleotide analog molecule and bases in a standard polynucleotide (e.g., single-stranded RNA or single-stranded DNA). Preferred analogs are those having a substantially uncharged, phosphorus containing backbone.

One method for producing antisense oligomers is the alkylation of the 2' hydroxyribose position and the incorporation of a phosphorothioate backbone to produce molecules that superficially resemble RNA but that are much more resistant to nuclease degradation, although persons skilled in the art of the invention will be aware of other forms of suitable backbones that may be useable in the objectives of the invention.

To avoid degradation of mRNA during duplex formation with the antisense oligomers, the antisense oligomers used in the method may be adapted to minimise or prevent cleavage of the target RNA by endogenous RNase H. This property is highly preferred, as the treatment of the RNA with the unmethylated oligomers, either intracellular or in crude extracts that contain RNase H, leads to degradation of the RNA strand of the pre-mRNA: antisense oligomer duplexes. Any form of modified antisense oligomers that is capable of by-passing or not inducing such degradation may be used in the present method. The nuclease resistance may be achieved by modifying the antisense oligomers of the invention so that it comprises partially unsaturated aliphatic hydrocarbon chain and one or more polar or charged groups including carboxylic acid groups, ester groups, and alcohol groups.

Antisense oligomers that do not activate RNase H can be made in accordance with known techniques. Such antisense oligomers that may be deoxyribonucleotide or ribonucleotide sequences, simply contain any structural modification that sterically hinders or prevents binding of RNase H to a duplex molecule containing the oligomer as one member thereof, which structural modification does not substantially hinder or disrupt duplex formation. Because the portions of the oligomer involved in duplex formation are substantially different from those portions involved in RNase H binding thereto, numerous antisense oligomers that do not activate RNase H are available. For example, such antisense oligomers may be oligomers wherein at least one, or all, of the inter-nucleotide bridging phosphate residues are modified phosphates, such as methyl phosphonates, methyl phosphorothioates, phosphoromorpholidates, phosphoropiperazidates boranophosphates, amide linkages and phosphoramidates. For example, every other one of the internucleotide bridging phosphate residues may be modified as described.

An example of antisense oligomers that when duplexed with RNA are not cleaved by cellular RNase H is 2'-O-methyl derivatives. Such 2'-O-methyl- oligoribonucleotides are stable in a cellular environment and in animal tissues, and their duplexes with RNA have higher Tm values than their ribo- or deoxyribo- counterparts. Alternatively, the nuclease resistant antisense oligomers of the invention may have at least one of the last 3'-terminus nucleotides fluoridated. Still alternatively, the nuclease resistant antisense oligomers of the invention have phosphorothioate bonds linking between at least two of the last 3-terminus nucleotide bases, preferably having phosphorothioate bonds linking between the last four 3'-terminal nucleotide bases.

While the antisense oligomers described above are a preferred form of the antisense oligomers of the present invention, the present invention includes other oligomeric antisense molecules, including but not limited to oligomer mimetics such as are described below.

Specific examples of preferred antisense oligomers useful in this invention include oligomers containing modified backbones or non-natural inter-nucleoside linkages. As defined in this specification, oligomers having modified backbones include those that retain a phosphorus atom in the backbone and those that do not have a phosphorus atom in the backbone. For the purposes of this specification, and as sometimes referenced in the art, modified oligomers that do not have a phosphorus atom in their inter-nucleoside backbone can also be antisense oligomers.

In some oligomer mimetics, both the sugar and the inter-nucleoside linkage, i.e., the backbone, of the nucleotide units are replaced with novel groups. The base units are maintained for hybridization with an appropriate nucleic acid target compound. One such oligomeric compound, an oligomer mimetic that has been shown to have excellent hybridization properties, is referred to as a peptide nucleic acid (PNA).

Another preferred chemistry is the phosphorodiamidate morpholino oligomer (PMO) oligomeric compounds that are not degraded by any known nuclease or protease. These compounds are uncharged, do not activate RNase H activity when annealed to an RNA strand and have been shown to exert sustained translational blocking after *in vivo* administration (Summerton and Weller, Antisense Nucleic Acid Drug Development, 7, 187-197).

Modified oligomers may also contain one or more substituted sugar moieties. Oligomers may also include nucleobase (often referred to in the art simply as "base") modifications or substitutions. Certain nucleobases are particularly useful for increasing the binding affinity of the oligomeric compounds of the invention.

Another modification of the oligomers of the invention involves chemically linking to the oligomer one or more moieties or conjugates that enhance the activity, cellular distribution or cellular uptake of the oligomer. Such moieties may include but are not limited to lipid moieties.

Cell penetrating peptides have been added to phosphorodiamidate morpholino oligomers (PMO) to enhance cellular uptake and nuclear localization. Different peptide tags have been shown to influence efficiency of uptake and target tissue specificity.

Vivo-morpholino is another example of the modifications to improve the cellular uptake and delivery where specific moieties have been added to PMOs.

Combination of antisense oligomers with the C-end Rule (CendR) peptides can also enhance the tissue delivery and efficiency of the cellular uptake of the oligomers.

Combination of antisense oligomers with the lipid nanoparticles can also be used to enhance the tissue delivery and efficiency of the cellular uptake of the oligomers.

It is not necessary for all positions in each compound to be uniformly modified, and in fact more than one of the modifications may be incorporated in a single compound or even at a single nucleoside within an oligomer. The present invention also includes antisense oligomers that are chimeric compounds. "Chimeric" antisense oligomers or "chimeras," in the context of this invention, are antisense oligomers, particularly oligomers that contain two or more chemically distinct regions, each made up of at least one monomer unit, i.e., a nucleotide in the case of an oligomer compound. These oligomers typically contain at least one region wherein the oligomer is modified to confer upon the oligomer or antisense oligomer increased resistance to nuclease degradation, increased cellular uptake, and an additional region for increased binding affinity for the target nucleic acid.

The activity of antisense oligomers and variants thereof can be assayed according to routine techniques in the art. For example, expression levels and splice forms of surveyed RNAs and proteins may be assessed by any of a wide variety of well-known methods for detecting expression levels and splice forms of a transcribed nucleic acid or protein. Non-limiting examples of such methods include RT-PCR of transcripts of RNA followed by size separation of PCR products, nucleic acid hybridization methods e.g., Northern blots and/or use of nucleic acid arrays; nucleic acid amplification methods; immunological methods for detection of proteins; protein purification methods; and protein function or activity assays.

RNA expression levels can be assessed by preparing mRNA or cDNA (i.e., a transcribed polynucleotide) from a sample, and by hybridizing the mRNA or cDNA with a complement of the assayed nucleic acid, or a fragment thereof. cDNA can, optionally, be amplified using any of a variety of polymerase chain reaction or in vitro transcription methods prior to hybridization with the complementary polynucleotide; preferably, it is not amplified. Expression of one or more transcripts can also be detected using reverse transcription followed by quantitative PCR to assess the level of expression of the transcript(s).

Genome-wide RNA expression level can be assessed using RNA sequencing technologies or RNA hybridization arrays. These assays are necessary to evaluate the activity of antisense oligomers and variants thereof on the transcriptome, changes in the transcriptome and transcript specific splicing effects.

The present invention provides antisense oligomer induced translational blocking of the inflammatory network genes, using clinically applicable oligomer chemistries and delivery systems to direct downregulation of the inflammatory network genes to therapeutic levels. Substantial reduction in the amount of the proteins from the inflammatory network genes transcription, are achieved by:
1) oligomer refinement *in vitro* using cell models, through experimental assessment of (i) mRNA translational target motifs, (ii) antisense oligomer length and development of oligomer cocktails, (iii) choice of chemistry, (iv) the addition of cell-penetrating peptides (CPP) and (v) the addition of CendR peptides or other moieties (Vivo-morpholino) to enhance oligomer delivery to cells and tissues; and
2) detailed evaluation of a novel approach to downregulate the inflammatory network genes.

As such, it is demonstrated herein that translational blocking of the inflammatory network genes with specific antisense oligomers. In this way functionally significant reduction of inflammatory network proteins of the inflammatory network can be obtained, thereby reducing the severe pathology associated with atopic dermatitis, psoriasis, and chronic inflammatory diseases.

The antisense oligomers used in accordance with this invention may be conveniently made through the well-known technique of solid phase synthesis. Equipment for such synthesis is sold by several vendors including, for example, Applied Biosystems (Foster City, Calif.).

Any other means for such synthesis known in the art may additionally or alternatively be employed.

The antisense oligomers of the invention are synthesised *in vitro* and include antisense compositions of biological origin, or genetic vector constructs designed to direct the *in vivo* synthesis of antisense oligomers. The molecules of the invention may also be mixed, encapsulated, conjugated, or otherwise associated with other molecules, molecule structures or mixtures of compounds, as for example, liposomes, receptor targeted molecules etc oral, rectal, topical, or other formulations, for assisting in uptake, distribution and/or absorption.

The antisense oligomers may be formulated for oral, topical, parenteral, or other delivery, particularly formulations for topical and injectable delivery. The formulations may be formulated for assisting in uptake, distribution and/or absorption at the site of delivery or activity.

### Method of Treatment

The antisense oligomers of the present invention also can be used as a prophylactic or therapeutic, which may be utilised for the purpose of treatment of a disease. Accordingly, in one embodiment the present invention provides antisense oligomers that bind to a selected target in the mRNA of the inflammatory network genes to block the translation of the RNA as described herein, in a therapeutically effective amount, admixed with a pharmaceutically acceptable carrier, diluent, or excipient.

According to a still further aspect of the invention, there is provided one or more antisense oligomers as described herein for use in an antisense oligomer-based therapy. Preferably, the therapy is for a condition related to the expression of inflammatory network genes. More preferably, the therapy for a condition related to the expression of inflammatory network genes is therapy for atopic dermatitis, psoriasis, and chronic inflammatory diseases.

More specifically, the antisense oligomer may be selected from Table 1, or the group consisting of any one or more of in SEQ ID NOs: 1-14, more preferably SEQ ID NOs: 1-3, 4-7, 9 and 14, even more preferably SEQ ID NOs: 1-3, 9 and 14, and combinations or cocktails thereof. This includes sequences which can hybridise to such sequences under stringent hybridisation conditions, sequences complementary thereto, sequences containing modified bases, modified backbones, and functional truncations or extensions thereof which possess mRNA translational blocking activity in the inflammatory network transcripts.

The invention extends also to a combination of two or more antisense oligomers capable of binding to a selected target to induce translational blocking activity in the inflammatory network transcripts. The combination may be a cocktail of two or more antisense oligomers, a construct comprising two or more antisense oligomers joined together for use in an antisense oligomer-based therapy.

There is therefore provided a method to treat, prevent or ameliorate the effects of a disease associated with inflammatory network gene expression, comprising the step of:
a) administring to the patient an effective amount of one or more antisense oligomers or pharmaceutical composition comprising one or more antisense oligomers as described herein.

Preferably the disease associated with inflammatory network transcripts expression in a patient is atopic dermatitis or psoriasis.

Therefore, the invention provides a method to treat, prevent or ameliorate the effects of atopic dermatitis or psoriasis, comprising the step of:
a) administring to the patient an effective amount of one or more antisense oligomers or pharmaceutical composition comprising one or more antisense oligomers as described herein.

Preferably, the therapy is used to suppress the inflammatory network transcripts and to downregulate the activation of these genes and transcripts. The downregulation of the inflammatory network transcripts is preferably achieved through mRNA translational blocking.

The suppression of the inflammatory network transcripts will preferably lead to a reduction in the quantity, duration, or severity of the symptoms of the inflammation-related condition or pathology, such as atopic dermatitis, psoriasis, or hidradenitis suppurativa (*acne inversa*)*.*

An "effective amount" or "therapeutically effective amount" refers to an amount of therapeutic compound, such as an antisense oligomer, administered to a mammalian subject, either as a single dose or as part of a series of doses that is effective to produce a desired therapeutic effect.

Treatment may be monitored, e.g., by general indicators of disease known in the art. As used herein, "treatment" of a subject (e.g., a mammal, such as a human) or a cell is any type of intervention used to alter the natural course of the individual or cell. Treatment includes, but is not limited to, administration of a pharmaceutical composition, and may be performed either prophylactically or after the initiation of a pathologic event or contact with an etiologic agent. Also included are "prophylactic" treatments that can be directed to reducing the rate of progression of the disease or condition being treated, delaying the onset of that disease or condition, or reducing the severity of its onset. "Treatment" or "prophylaxis" does not necessarily indicate complete eradication, cure, or prevention of the disease or condition, or associated symptoms thereof.

A "subject" as used herein, includes any animal that exhibits a symptom, or is at risk for exhibiting a symptom that can be treated with an antisense compound of the invention, or any of the symptoms associated with these conditions. The subject with the disease associated with the activation of the inflammatory network transcripts may be a mammal, including a human. Other suitable subjects include laboratory animals (such as mouse, rat, rabbit, or guinea pig), farm animals, and domestic animals or pets (such as a cat or dog). Non-human primates and, preferably, human subjects, are included.

The antisense oligomers of the present invention may also be used in conjunction with alternative therapies, such as drug therapies.

The present invention therefore provides a method of treating, preventing, or ameliorating the effects of a disease or condition associated with the increased expression of the inflammatory network gene transcripts, wherein the antisense oligomers of the present invention and administered sequentially or concurrently with another alternative therapy associated with treating, preventing, or ameliorating the effects of a disease or condition associated with the increased expression of the inflammatory network gene transcripts. Preferably, the disease or condition is atopic dermatitis, psoriasis, or other chronic inflammatory diseases.

### Delivery

The antisense oligomers of the present invention also can be used as a prophylactic or therapeutic that may be utilised for the purpose of treatment of a disease. Accordingly, in one embodiment the present invention provides antisense oligomers that bind to a selected target in the inflammatory network gene transcripts mRNA to induce efficient and consistent translational blocking as described herein, in a therapeutically effective amount, admixed with a pharmaceutically acceptable carrier, diluent, or excipient.

There is also provided a pharmaceutical, prophylactic, or therapeutic composition to treat, prevent or ameliorate the effects of a disease related to activation the inflammatory network gene transcripts in a patient, the composition comprising:
a) one or more antisense oligomers as described herein; and
b) one or more pharmaceutically acceptable carriers and/or diluents.

In one embodiment, the antisense oligomer is administered in an amount and manner effective to result in a peak blood concentration of at least 500 nM antisense oligomer. Typically, one or more doses of antisense oligomer are administered, initially at regular intervals as a loading dose for a period of about one to two weeks or months and then as required to maintain clinically therapeutic levels. Preferred doses for topical administration are from about 0.01 mg to 200 mg oligomer per kg. Preferred doses for oral administration are from about 0.01 mg to 200 mg oligomer per kg. For intravenous administration, preferred doses are from about 0.5 mg to 100 mg oligomer per kg. For intravenous or subcutaneous administration, the antisense oligomer may be administered at a dosage of about up to 200 mg/kg weekly.

The antisense oligomer may be administered at regular intervals for a short time, e.g., daily. However, in many cases the oligomer is administered intermittently over a longer period. Administration may be followed by, or concurrent with, administration of a small molecule drug or other therapeutic treatment. The treatment regimen may be adjusted as indicated, based on the results of immunoassays, biomarker assays other biochemical tests and physiological examination of the subject under treatment.

Dosing may be dependent on severity and responsiveness of the disease state to be treated, with the course of treatment lasting from several days to years, or until a cure is reached or a diminution of the disease state is achieved. Alternatively, dosing may be titrated against disease progression rate. A baseline progression is expected to be established on a case-by-case basis. Then the progression rate after an initial once off dose is monitored to check that there is a reduction in the rate. Preferably, there is no progression after dosing. Successful treatment preferably results in no further progression of the disease or even some recovery. Optimal dosing schedules can be calculated from measurements of drug accumulation in the body of the patient. Persons of ordinary skill can easily determine optimum dosages, dosing methodologies and repetition rates.

Optimum dosages may vary depending on the relative potency of individual oligomers and can generally be estimated based on EC50s found to be effective in *in vitro* and *in vivo* animal studies.

In general, dosage between 10 pg to 0.2 g per kg of body weight, and may be given once or more daily, weekly, monthly, or yearly, or even less frequently. Persons of ordinary skill in the art can easily estimate repetition rates for dosing based on measured residence times and concentrations of the drug in bodily fluids or tissues. Following successful treatment, it may be desirable to have the subject undergo maintenance therapy to prevent the recurrence of the disease state, wherein the oligomer is administered in maintenance doses, ranging from 10 pg to 0.2 g per kg of body weight, once or more daily, to once every 20 years.

An effective *in vivo* treatment regimen using the antisense oligomers of the invention may vary according to the duration, dose, frequency and route of administration, as well as the condition of the subject under treatment for atopic dermatitis, psoriasis, or other chronic inflammatory diseases. Accordingly, such *in vivo* therapy will often require monitoring by tests appropriate to the disorder under treatment, and corresponding adjustments in the dose or treatment regimen, to achieve an optimal therapeutic outcome.

Treatment may be monitored by general indicators of disease known in the art. The efficacy of an in vivo administered antisense oligomers of the invention may be determined from biological samples taken from a subject prior to, during and after administration of the antisense oligomer. Assays of such samples include:
(1) monitoring the presence or absence of heteroduplex formation with target and non target sequences, using procedures known to those skilled in the art, e.g., an electrophoretic gel mobility assay;
(2) monitoring the amount of a mRNA or protein in relation to a reference normal mRNA or protein as determined by standard techniques such as RT-PCR, Northern blotting, ELISA or Western blotting.

Delivery is a major challenge for antisense oligomers. Different cell-penetrating peptides (CPP) localize PMOs to varying degrees in different conditions and cell lines and tissues, and novel CPPs have been evaluated by the inventors for their ability to deliver PMOs to the target cells. The terms CPP or "a peptide moiety that enhances cellular uptake" are used interchangeably and refer to cell penetrating peptides, also called "transport peptides", "carrier peptides", or "peptide transduction domains". Lipid nanoparticles, CendR peptides or any other delivery systems can be used to enhance the delivery of oligomers.

The present invention therefore provides antisense oligomers of the present invention in combination with cell-penetrating peptides for manufacturing therapeutic pharmaceutical compositions.

Vivo-morpholino is another example for the efficient delivery modifications.

The present invention therefore provides antisense oligomers of the present invention in combination with lipid nanoparticles (LNP) for manufacturing therapeutic pharmaceutical compositions.

### Excipients

In a form of the invention there are provided pharmaceutical compositions comprising therapeutically effective amounts of one or more antisense oligomers of the invention together with pharmaceutically acceptable diluents, preservatives, solubilizers, emulsifiers, adjuvants, and/or carriers. Such compositions include diluents of various buffer content, pH and ionic strength and additives such as detergents and solubilizing agents, anti-oxidants, preservatives and bulking substances. The material may be incorporated into particulate preparations of polymeric compounds such as polylactic acid, polyglycolic acid, etc. or into liposomes. Hylauronic acid may also be used. Such compositions may influence the physical state, stability, rate of in vivo release, and rate of in vivo clearance of the present proteins and derivatives. The compositions may be prepared in liquid form, or may be in dried powder, such as a lyophilised form.

The antisense oligomers of the present invention are preferably delivered in a pharmaceutically acceptable composition.

The present invention further provides one or more antisense oligomers adapted to aid in the prophylactic or therapeutic treatment, prevention, or amelioration of symptoms of a disease such as the inflammatory network genes related disease or pathology in a form suitable for delivery to a patient.

The phrase "pharmaceutically acceptable" refers to molecular entities and compositions that are physiologically tolerable and do not typically produce an allergic or similarly untoward reaction, such as gastric upset and the like, when administered to a patient. The term "carrier" refers to a diluent, adjuvant, excipient, or vehicle with which the compound is administered. Such pharmaceutical carriers can be sterile liquids, such as water and oils, including those of petroleum, animal, vegetable or synthetic origin, such as peanut oil, soybean oil, mineral oil, sesame oil and the like. Water or saline solutions and aqueous dextrose and glycerol solutions are preferably employed as carriers, particularly for injectable solutions.

In a more specific form of the invention there are provided pharmaceutical compositions comprising therapeutically effective amounts of one or more antisense oligomers of the invention together with pharmaceutically acceptable diluents, preservatives, solubilizers, emulsifiers, adjuvants, and/or carriers. Such compositions include diluents of various buffer content, pH and ionic strength and additives such as detergents and solubilizing agents, anti-oxidants, preservatives and bulking substances. The material may be incorporated into particulate preparations of polymeric compounds such as polylactic acid, polyglycolic acid, etc. or into liposomes. Hylauronic acid may also be used. Such compositions may influence the physical state, stability, rate of in vivo release, and rate of in vivo clearance of the present proteins and derivatives. The compositions may be prepared in liquid form, or may be in dried powder, such as a lyophilised form.

It will be appreciated that pharmaceutical compositions provided according to the present invention may be administered by any means known in the art. The pharmaceutical compositions for administration are administered by injection, orally, topically or by the pulmonary or nasal route. For example, the antisense oligomers may be delivered by intravenous, intra-arterial, intraperitoneal, intramuscular or subcutaneous routes of administration. The appropriate route may be determined by one of skill in the art, as appropriate to the condition of the subject under treatment. Vascular or extravascular circulation, the blood or lymph system, and the cerebrospinal fluid are some non-limiting sites where the antisense oligomer may be introduced. Direct CNS delivery may be employed, for instance, intracerebral ventricular or intrathecal administration may be used as routes of administration.

Formulations for topical administration include those in which the oligomers of the disclosure are in admixture with a topical delivery agent such as lipids, liposomes, fatty acids, fatty acid esters, steroids, chelating agents and surfactants. Lipids and liposomes include neutral negative and cationic. For topical or other administration, oligomers of the disclosure may be encapsulated within liposomes or may form complexes thereto, especially to cationic liposomes. Alternatively, oligomers may be complexed to lipids, especially to cationic lipids. Fatty acids and esters, pharmaceutically acceptable salts thereof.

In certain embodiments, the antisense oligomers of the disclosure can be delivered by topical or transdermal methods including delivery to skin surfaces. Such topical or transdermal and emulsion/liposome-mediated methods of delivery are described for delivery of antisense oligomers in the art.

The antisense oligomers described herein may also be delivered via an implantable device. Design of such a device is an art-recognized process. Preferably the implant can be implanted into the body for sustained delivery of the antisense oligomers.

The pharmaceutical formulations of the present invention that may conveniently be presented in unit dosage form, may be prepared according to conventional techniques well known in the pharmaceutical industry. Such techniques include the step of bringing into association the active ingredients with the pharmaceutical carrier(s) or excipient(s). In general, the formulations are prepared by uniformly and intimately bringing into association the active ingredients with liquid carriers or finely divided solid carriers or both, and then, if necessary, shaping the product.

### Use

According to another aspect of the invention there is provided the use of one or more antisense oligomers as described herein in the manufacture of a medicament for the modulation or control of a disease associated with activated expression of the inflammatory network gene transcripts.

The invention also provides for the use of purified and isolated antisense oligomers as described herein, for the manufacture of a medicament for treatment of a disease associated with activated expression of the inflammatory network gene transcripts.

There is also provided the use of purified and isolated antisense oligomers as described herein, for the manufacture of a medicament to treat, prevent or ameliorate the effects of a disease associated with activated expression of the inflammatory network gene transcripts.

Preferably, the inflammatory network activation-related pathology or diseases are atopic dermatitis or psoriasis.

The invention extends, according to a still further aspect thereof, to cDNA or cloned copies of the antisense oligomer sequences of the invention, as well as to vectors encoding the antisense oligomer sequences of the invention. The invention extends further also to cells containing such sequences and/or vectors.

### Kits

There is also provided a kit to treat, prevent or ameliorate the effects of a diseases associated with inflammatory network gene transcript expression in a patient, which kit comprises at least an antisense oligomer as described herein and combinations or cocktails thereof, packaged in a suitable container, together with instructions for its use.

In a preferred embodiment, the kits will contain at least one antisense oligomer as described herein or as shown in Table 1, or in SEQ ID NOs: 1-14, more preferably SEQ ID NOs: 1-3, 4-7, 9 and 14, even more preferably SEQ ID NOs: 1-3, 9 and 14, or a cocktail of antisense oligomers, as described herein. The kits may also contain peripheral reagents such as buffers, stabilizers, etc.

There is therefore provided a kit to treat, prevent or ameliorate a disease or condition associated with inflammatory network gene transcript expression in a patient, which kit comprises at least an antisense oligomer described herein or as shown in Table 1 and combinations or cocktails thereof, packaged in a suitable container, together with instructions for its use.

There is also provided a kit to treat, prevent or ameliorate a disease or condition associated with inflammatory network gene transcript expression in a patient, which kit comprises at least an antisense oligomer selected from the group consisting of any one or more of in SEQ ID NOs: 1-14, more preferably SEQ ID NOs: 1-3, 4-7, 9 and 14, even more preferably SEQ ID NOs: 1-3, 9 and 14, and combinations or cocktails thereof, packaged in a suitable container, together with instructions for its use.

Preferably, the inflammatory network activation-related pathologies or diseases are atopic dermatitis or psoriasis.

The contents of the kit can be lyophilized, and the kit can additionally contain a suitable solvent for reconstitution of the lyophilized components. Individual components of the kit would be packaged in separate containers and associated with such containers, can be a notice in the form prescribed by a governmental agency regulating the manufacture, use or sale of pharmaceuticals or biological products, which notice reflects approval by the agency of manufacture, use or sale for human administration.

When the components of the kit are provided in one or more liquid solutions, the liquid solution can be an aqueous solution, for example a sterile aqueous solution. For in vivo use, the expression construct may be formulated into a pharmaceutically acceptable composition for syringe. In this case the container means may itself be an inhalant, syringe, pipette, eye dropper, or other such like apparatus, from which the formulation may be applied to an affected area of the animal, such as the lungs, injected into an animal, or even applied to and mixed with the other components of the kit.

In an embodiment, the kit of the present invention comprises a composition comprising a therapeutically effective amount of an antisense oligomer capable of binding to a selected target on the inflammatory network gene transcripts to modify mRNA translation of the inflammatory network gene transcripts or part thereof. In an alternative embodiment, the formulation is in pre-measured, premixed and/or prepackaged. Preferably, the solution is sterile.

The kit of the present invention may also include instructions designed to facilitate user compliance. Instructions, as used herein, refers to any label, insert, etc., and may be positioned on one or more surfaces of the packaging material, or the instructions may be provided on a separate sheet, or any combination thereof. For example, in an embodiment, the kit of the present invention comprises instructions for administering the formulations of the present invention. In one embodiment, the instructions indicate that the formulation of the present invention is suitable for the treatment of psoriasis Such instructions may also include instructions on dosage, as well as instructions for administration via injection, orally, topically or by the pulmonary or nasal route.

The antisense oligomers and suitable excipients can be packaged individually so to allow a practitioner or user to formulate the components into a pharmaceutically acceptable composition as needed. Alternatively, the antisense oligomers and suitable excipients can be packaged together, thereby requiring minimal formulation by the practitioner or user. In any event, the packaging should maintain chemical, physical, and aesthetic integrity of the active ingredients.

### General

Those skilled in the art will appreciate that the invention described herein is susceptible to variations and modifications other than those specifically described. The invention includes all such variation and modifications. The invention also includes all of the steps, features, formulations and compounds referred to or indicated in the specification, individually or collectively and any and all combinations or any two or more of the steps or features.

The present invention is not to be limited in scope by any of the specific embodiments described herein. These embodiments are intended for the purpose of exemplification only. Functionally equivalent products, formulations and methods are clearly within the scope of the invention as described herein.

The invention described herein may include one or more range of values (eg. Size, displacement and field strength etc). A range of values will be understood to include all values within the range, including the values defining the range, and values adjacent to the range which lead to the same or substantially the same outcome as the values immediately adjacent to that value which defines the boundary to the range. Accordingly, unless indicated to the contrary, the numerical parameters set forth in the specification and claims are approximations that may vary depending upon the desired properties sought to be obtained by the present invention.

Throughout this specification, unless the context requires otherwise, the word "comprise" or variations such as "comprises" or "comprising", will be understood to imply the inclusion of a stated integer or group of integers but not the exclusion of any other integer or group of integers.

Other definitions for selected terms used herein may be found within the detailed description of the invention and apply throughout. Unless otherwise defined, all other scientific and technical terms used herein have the same meaning as commonly understood to one of ordinary skill in the art to which the invention belongs. The term "active agent" may mean one active agent, or may encompass two or more active agents.

## Claims

1. An isolated or purified antisense oligomer with a modified backbone structure for blocking specific mRNA translation of the specific member of the inflammatory network gene transcripts or part thereof, wherein said antisense oligomer is selected from sequences SEQ ID NOs: 1 to 14.

2. The antisense oligomer of claim 1 wherein the antisense oligomer contains one or more nucleotide positions subject to an alternative chemistry or modification chosen from the list comprising: (i) modified sugar moieties; (ii) resistance to RNase H; (iii) oligomeric mimetic chemistry.

3. The antisense oligomer of claim 1 wherein the antisense oligomer is further modified by:
(i) chemical conjugation to a moiety; and/or (ii) tagging with a cell penetrating peptide.

4. The antisense oligomer of claim 1 wherein the antisense oligomer is a phosphorodiamidate morpholino oligomer.

5. The antisense oligomer of claim 1 wherein when any uracil (U) is present in the nucleotide sequence, the uracil (U) is replaced by a thymine (T).

6. The antisense oligomer of claim 1 that operates to induce translational blocking of the inflammatory network gene transcripts or part thereof.

7. A method for manipulating the expression of the inflammatory network gene transcripts, the method including the step of:
a) providing an antisense oligomer according to any one of claims 1 to 6 and combinations or cocktails thereof and allowing the oligomer(s) to bind to a target nucleic acid site.

8. A pharmaceutical, prophylactic, or therapeutic composition to treat, prevent or ameliorate the effects of a disease related to inflammatory network gene transcript expression in a patient, the composition comprising:
a) an antisense oligomer according to any one of claims 1 to 6 and combinations or cocktails thereof, and
b) one or more pharmaceutically acceptable carriers and/or diluents.

9. A method of treatment, of prevention or of amelioration the effects of disease associated with the inflammatory network gene transcripts, comprising the step of:
a) administering to the patient an effective amount of an antisense oligomer, or pharmaceutical composition comprising an antisense oligomer, according to any one of claims 1 to 7 and combinations or cocktails thereof.

10. The use of an antisense oligomer according to any one of claims 1 to 6 and combinations or cocktails thereof, for the manufacture of a medicament to treat, prevent or ameliorate the effects of a disease associated with the expression of the inflammatory network gene transcripts.

11. A kit to treat, prevent or ameliorate the effects of a disease associated with inflammatory network gene expression in a patient, which kit comprises at least an antisense oligomer according to any one of claims 1 to 6 and combinations or cocktails thereof, packaged in a suitable container, together with instructions for its use.

12. The composition of claim 8, method of claim 7 or 9, use of claim 11 or kit of claim 11 wherein the inflammatory network gene transcripts expression related disease is atopic dermatitis, psoriasis, or any other chronic inflammatory condition.

13. The composition of claim 8, method of claim 7 or 9, use of claim 11 or kit of claim 11 wherein the subject with the disease associated with inflammatory network gene transcripts expression is a human.

14. The antisense oligomer of claim 1 selected from the list comprising: SEQ ID NOs: 1-3, 4-7, 9 and 14.

15. The antisense oligomer of claim 1 selected from the list comprising: SEQ ID NOs: 1-3, 9 and 14.
